(19) Europäisches Patentamt
European Patent Office
Office européen des brevets

(11) EP 0 970 103 B1

(12) # EUROPÄISCHE PATENTSCHRIFT

(45) Veröffentlichungstag und Bekanntmachung des Hinweises auf die Patenterteilung:
**17.04.2002 Patentblatt 2002/16**

(21) Anmeldenummer: **98905419.2**

(22) Anmeldetag: **09.02.1998**

(51) Int Cl.[7]: **C07J 41/00**, A61K 31/56, C07J 53/00, C07J 1/00, C07J 43/00

(86) Internationale Anmeldenummer:
**PCT/EP98/00752**

(87) Internationale Veröffentlichungsnummer:
**WO 98/34947 (13.08.1998 Gazette 1998/32)**

(54) **ANTIGESTAGEN WIRKSAME STEROIDE MIT FLUORIERTER 17ALPHA-ALKYLKETTE**
ANTIGESTAGENIC STEROIDS WITH A FLUORINATED 17ALPHA-ALKYL CHAIN
STEROIDES A ACTIVITE ANTIGESTAGENE A CHAINE 17ALPHA-ALKYLE FLUOREE

(84) Benannte Vertragsstaaten:
**AT BE CH DE DK ES FI FR GB GR IE IT LI LU MC NL PT SE**
Benannte Erstreckungsstaaten:
**AL LT LV RO SI**

(30) Priorität: **07.02.1997 DE 19706061**

(43) Veröffentlichungstag der Anmeldung:
**12.01.2000 Patentblatt 2000/02**

(73) Patentinhaber: **Schering Aktiengesellschaft**
**13353 Berlin (DE)**

(72) Erfinder:
- **SCHWEDE, Wolfgang**
  **D-13467 Berlin (DE)**
- **CLEVE, Arwed**
  **D-10707 Berlin (DE)**
- **KLAR, Ulrich**
  **D-13503 Berlin (DE)**
- **NEEF, Günter**
  **D-10711 Berlin (DE)**
- **CHWALISZ, Kristof**
  **D-13503 Berlin (DE)**
- **SCHNEIDER, Martin**
  **D-13469 Berlin (DE)**
- **FUHRMANN, Ulrike**
  **D-10587 Berlin (DE)**
- **HESS-STUMPP, Holger**
  **D-13347 Berlin (DE)**

(56) Entgegenhaltungen:
**EP-A- 0 245 170**
**EP-A- 0 299 913**
**EP-A- 0 369 881**
**DE-A- 3 844 408**

- **CHEMICAL ABSTRACTS, vol. 123, no. 21, 20.November 1995 Columbus, Ohio, US; abstract no. 286388, WANG Z ET AL: "Preparation of trifluoromethyl steroids as postcoital contraceptives" XP002065659 & CN 1 100 729 A (SHANGHAI INSTITUTE OF ORGANIC CHEMISTRY, CHINESE ACADEMY OF SCIENCES;P) 29.März 1995 & THE AMERICAN CHEMICAL SOCIETY: "CHEMICAL ABSTRACTS FORMULA INDEX, VOLUME 123, C26 H28 BR N3 O3-Z" 1995 , CHEMICAL ABSTRACTS SERVICE , COLUMBUS OHIO US**
- **WANG Z ET AL: "Trifluoromethylation of steroidal ketones" JOURNAL OF FLUORINE CHEMISTRY., Bd. 69, Nr. 1, Oktober 1994, LAUSANNE CH, Seiten 1-3, XP002065658**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 970 103 B1

## Beschreibung

Die vorliegende Erfindung betrifft antigestagen wirksame Steroide mit einer fluorierten 17α-Alkylkette, Verfahren zu ihrer Herstellung, pharmazeutische Präparate, die diese enthalten sowie ihre Verwendung zur Herstellung von Arzneimitteln.

Die Erfindung betrifft 17α-Fluoralkylsteroide der allgemeinen Formel I

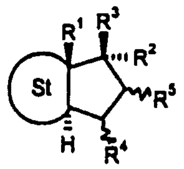

I

worin

$R^1$ für eine Methyl- oder Ethylgruppe,

$R^2$ für einen Rest der Formel $C_nF_mH_o$, wobei n 2, 3, 4, 5 oder 6, m>1 und m+o = 2n+1 ist,

$R^3$ für eine freie, veretherte oder veresterte Hydroxygruppe,

$R^4$ und $R^5$ je für ein Wasserstoffatom, gemeinsam für eine zusätzliche Bindung oder eine Methylengruppe,

St für ein steroidales ABC-Ringsystem der Teilformel A, B oder C

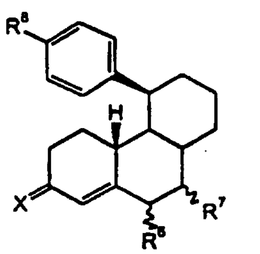

A

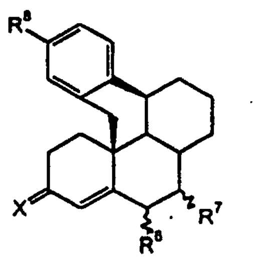

B

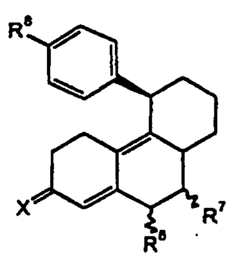

C

stehen,
worin

$R^6$ ein Wasserstoffatom, eine geradkettige $C_1$-$C_4$- oder verzweigte $C_3$-$C_4$-Alkylgruppe oder ein Halogenatom,

$R^7$ ein Wasserstoffatom, eine geradkettige $C_1$-$C_4$- oder eine verzweigte $C_3$-$C_4$-Alkylgruppe, oder, wenn St für ein steroidales ABC-Ringsystem A oder B steht, außerdem $R^6$ und $R^7$ gemeinsam eine zusätzliche Bindung,

X ein Sauerstoffatom, eine Hydroxyiminogruppierung =N~OH oder zwei Wasserstoffatome,

$R^8$ einen Rest Y oder einen gegebenenfalls mehrfach mit einer Gruppe Y substituierten Arylrest, wobei Y ein Wasserstoffatom, ein Halogenatom, eine -OH, $-NO_2$, $-N_3$, -CN, $-NR^{9a}R^{9b}$, $-NHSO_2R^9$, $-CO_2R^9$, $C_1$-$C_{10}$-Alkyl-, $C_1$-$C_{10}$-Alkoxy-, $C_1$-$C_{10}$-Alkanoyloxy-, Benzoyloxy-, $C_1$-$C_{10}$-Alkanoyl-, $C_1$-$C_{10}$-Hydroxyalkyl- oder Benzoylgruppe ist und $R^{9a}$ und $R^{9b}$ gleich oder verschieden sind und wie $R^9$ ein Wasserstoffatom oder eine $C_1$-$C_{10}$-Alkylgruppe darstellen,

bedeuten,

sowie für die Reste-$NR^{9a}R^{9b}$ auch deren physiologisch verträgliche Salze mit Säuren und für die Reste -$CO_2R^9$ mit $R^9$ in der Bedeutung von Wasserstoff auch deren physiologisch verträgliche Salze mit Basen.

Die für die Reste $R^6$ und $R^7$ eingezeichneten Schlangenlinien bedeuten, daß der betreffende Substituent α- oder β-ständig sein kann.

Bei den im Rahmen vorliegender Erfindung als $R^6$ und $R^7$ erwähnten Alkylgruppen handelt es sich um die Methyl-, Ethyl-, n- oder iso-Propyl-, n-, iso- oder tert.-Butylgruppe.
Bei den anderen $C_1$-$C_{10}$-Alkylgruppen Y, $R^9$, $R^{9a}$, $R^{9b}$ kommen noch die höheren Homologen wie beispielsweise die Pentyl-, neo-Pentyl-, Hexyl- bis Decylgruppe hinzu.
Unter $C_1$-$C_{10}$-Alkylgruppen sollen aber auch carbocyclische oder Alkylcycloalkyl-Gruppen mit bis zu 10 Kohlenstoffatomen, beispielsweise der Cyclopropyl-, Cyclopentyl-, Cycloheptyl-, Methylcyclopropyl-, Methylcyclopentyl- oder Methylcyclohexylrest, verstanden werden.
Eine Methyl- oder Ethylgruppe ist für alle vorstehenden Fälle bevorzugt.

$C_1$-$C_{10}$-Alkoxygruppen sind die um ein Sauerstoffatom verlängerten, von den vorstehend genannten Alkylgruppen abgeleiteten Reste, also z.B. der Methoxy-, Ethoxy-, n- oder iso-Propoxy-, n-, iso- oder tert.-Butoxyrest.

Unter $C_1$-$C_{10}$-Alkanoyl werden die Acylreste der geradkettigen und verzweigten $C_1$-$C_{10}$-Alkancarbonsäuren verstanden, also beispielsweise der Formyl-, Acetyl-, Propionyl-, Butyryloder iso-Butyrylrest etc..

$C_1$-$C_{10}$-Alkanoyloxyreste sind die um ein Sauerstoffatom verlängerten Reste der vorstehenden Alkanoylreste, also z.B. der Acetyloxy-, Propionyloxy-, Butyryloxyrest.

Sofern ein Halogenatom als Substituent erwähnt ist, kommt hierfür ein Fluor-, Chlor oder Bromatom infrage. Fluor ist bevorzugt.

Für die Reste $R^2$ sind perfluorierte Seitenketten der Länge n=2-4 zu bevorzugen und unter diesen ist wiederum die Pentafluorethyleinheit besonders zu bevorzugen.

$R^3$ steht in erster Linie für eine freie Hydroxygruppe.
Im Falle einer veretherten oder veresterten Hydroxygruppe als 17β-Substituent ist diese vorzugsweise mit einer $C_1$-$C_{10}$-Alkylgruppe verethert oder einer $C_1$-$C_{10}$-Alkanoylgruppe verestert. Für diese Alkyl- bzw. Alkanoylgruppe gelten die gleichen Bedeutungen wie vorstehend. Die Veretherung bzw. Veresterung der Hydroxygruppe erfolgt nach dem Fachmann geläufigen Methoden.

$R^4$ und $R^5$ stehen bevorzugterweise je für ein Wasserstoffatom oder gemeinsam für eine zusätzliche Bindung.

Ist $R^8$ eine Gruppe Y, so ist dies vorzugsweise eine $C_1$-$C_{10}$-Alkanoyl- oder (1-Hydroxy)-$C_1$-$C_{10}$-Alkylgruppe, wobei unter diesen Resten die Acetyl- und die Propionylgruppe besonders zu bevorzugen sind.

Bevorzugte carbocyclische oder heterocyclische Arylreste sind Phenyl, 1- oder 2-Naphthalinyl, 2- oder 3-Furanyl, 2- oder 3-Benzofuranyl, 2- oder 3-Thienyl, 2-, 3- oder 4-Pyridinyl. Als substituierte Arylreste $R^8$ sind in erster Linie der 4-Cyanphenyl- und ein 4-Halogenphenyl-, insbesondere der 4-Fluorphenylrest zu nennen.

Unter allen für $R^8$ als bevorzugt genannten Resten ist $R^8$ in der Bedeutung von Y und Y gleich Acetyl wiederum besonders zu bevorzugen.

Die nachstehend genannten Verbindungen sind erfindungsgemäß insbesondere bevorzugt:

11β-(4-Acetylphenyl)-17β-hydroxy-17α-(1,1,2,2,2-pentafluorethyl)estr-4-en-3-on;

4'-[17β-Hydroxy-3-oxo-17α-(1,1,2,2,2-pentafluorethyl)estr-4-en-11β-yl][1,1'-biphenyl]-4-carbonitril;

11β-(4'-Fluor[1,1'-biphenyl]-4-yl)-17β-hydroxy-17α-(1,1,2,2,2-pentafluorethyl)estr-4-en-3-on;

17β-Hydroxy-17α-(1,1,2,2,2-pentafluorethyl)-11β-[4-(3-pyridinyl)phenyl]estr-4-en-3-on;

11β-(4-Acetylphenyl)-17β-hydroxy-17α-(1,1,2,2,2-pentafluorethyl)estra-4,15-dien-3-on;

4'-[17β-Hydroxy-3-oxo-17α-(1,1,2,2,2-pentafluorethyl)estra-4,15-dien-11β-yl][1,1'-biphenyl]-4-carbonitril;

11β-(4'-Fluor[1,1'-biphenyl]-4-yl)-17β-hydroxy-17α-(1,1,2,2,2-pentafluorethyl)estra-4,15-dien-3-on;

17β-Hydroxy-17α-(1,1,2,2,2-pentafluorethyl)-11β-[4-(3-pyridinyl)phenyl]estra-4,15-dien-3-on;

11β-(4-Acetylphenyl)-17β-hydroxy-17α-(1,1,2,2,2-pentafluorethyl)estra-4,9-dien-3-on;

4'-[17ß-Hydroxy-3-oxo-17α-(1,1,2,2,2-pentafluorethyl)estra-4,9-dien-11β-yl][1,1'-biphenyl]-4-carbonitril;

11β-(4'-Fluor[1,1'-biphenyl]-4-yl)-17β-hydroxy-17α-(1,1,2,2,2-pentafluorethyl)estra-4,9-dien-3-on;

17β-Hydroxy-17α-(1,1,2,2,2-pentafluorethyl)-11β-[4-(3-pyridinyl)phenyl]estra-4,9-dien-3-on;

11 β-(4-Acetylphenyl)-17β-hydroxy-17α-(1,1,2,2,2-pentafluorethyl)estra-4,9,15-trien-3-on;

4'-[17β-Hydroxy-3-oxo-17α-(1,1,2,2,2-pentafluorethyl)estra-4,9,15-trien-11β-yl][1,1'-biphenyl]-4-carbonitril;

11 β-(4'-Fluor[1,1'-biphenyl]-4-yl)-17β-hydroxy-17α-(1,1,2,2,2-pentafluorethyl))estra-4,9,15-trien-3-on;

17β-Hydroxy-17α-(1,1,2,2,2-pentafluorethyl)-11β-[4-(3-pyridinyl)phenyl]estra-4,9,15-trien-3-on;

6'-Acetyl-9,11 α-dihydro-17β-hydroxy-17α-(1,1,2,2,2-pentafluorethyl)-4'*H*naphth[3',2',1':10,9,11]estr-4-en-3-on;

4-[9,11α-dihydro-17β-hydroxy-3-oxo-17α-(1,1,2,2,2-pentafluorethyl)-4'*H*naphth[3',2',1':10,9,11]estr-4-en-6'-yl] benzonitril;

9,11α-Dihydro-6'-(4-fluorphenyl)-17β-hydroxy-17α-(1,1,2,2,2-pentafluorethyl)-4'*H*naphth[3',2',1':10,9,11]estr-4-en-3-on;

9,11α-Dihydro-17β-hydroxy-17α-(1,1,2,2,2-pentafluorethyl)-6'-(3-pyridinyl)-4'*H*naphth[3',2',1':10,9,11]estr-4-en-3-on;

6'-Acetyl-9,11 α-dihydro-17β-hydroxy-17α-(1,1,2,2,2-pentafluorethyl)-4'*H*naphth[3',2',1':10,9,11]estra-4,15-dien-3-on;

4-[9,11α-dihydro-17β-hydroxy-3-oxo-17α-(1,1,2,2,2-pentafluorethyl)-4'*H*naphth[3',2',1':10,9,11]estra-4,15-dien-6'-yl]benzonitril;

9,11 α-Dihydro-6'-(4-fluorphenyl)-17β-hydroxy-17α-(1,1,2,2,2-pentafluorethyl)-4'*H*-naphth[3',2',1':10,9,11]estra-4,15-dien-3-on;

9,11α-Dihydro-17β-hydroxy-17α-(1,1,2,2,2-pentafluorethyl)-6'-(3-pyridinyl)-4'*H*naphth[3',2',1':10,9,11]estra-4,15-dien-3-on;

17β-Hydroxy-11β-(4-hydroxyphenyl)-17α-(1,1,2,2,2-pentafluorethyl)estra-4,9-dien-3-on;

17β-Hydroxy-11β-(4-hydroxyphenyl)-17α-(1,1,2,2,2-pentafluorethyl)estr-4-en-3-on;

9,11α-Dihydro-6',17β-dihydroxy-17α-(1,1,2,2,2-pentafluorethyl)-4'*H*naphth[3',2',1':10,9,11]estr-4-en-3-on;

11β-[4-(Acetyloxy)phenyl]-17β-hydroxy-17α-(1,1,2,2,2-pentafluorethyl)estra-4,9-dien-3-on;

11β-[4-(Acetyloxy)phenyl]-17β-hydroxy-17α-(1,1,2,2,2-pentafluorethyl)estr-4-en-3-on

6'-(Acetyloxy)-9,11α-dihydro-17β-hydroxy-17α-(1,1,2,2,2-pentafluorethyl)-4'*H*naphth[3',2',1':10,9,11]estr-4-en-3-on;

17β-Hydroxy-11β-[4-(hydroxymethyl)phenyl]-17α-(1,1,2,2,2-pentafluorethyl)estra-4,9-dien-3-on;

17β-Hydroxy-11β-[4-(hydroxymethyl)phenyl]-17α-(1,1,2,2,2-pentafluorethyl)estr-4-en-3-on;

9,11α-Dihydro-17β-hydroxy-6'-(hydroxymethyl)-17α-(1,1,2,2,2-pentafluorethyl)-4'*H*naphth[3',2',1':10,9,11]estr-4-en-3-on;

4-[17β-Hydroxy-3-oxo-17α-(1,1,2,2,2-pentafluorethyl)estra-4,9-dien-11β-yl]benzaldehyd;

4-[17β-Hydroxy-3-oxo-17α-(1,1,2,2,2-pentafluorethyl)estr-4-en-1β-yl]benzaldehyd;

9,11α-Dihydro-17β-hydroxy-3-oxo-17α-(1,1,2,2,2-pentafluorethyl)-4'*H*naphth[3',2',1':10,9,11]estr-4-en-6'-al;

4-[17β-Hydroxy-3-oxo-17α-(1,1,2,2,2-pentafluorethyl)estra-4,9-dien-11β-yl]benzoesäuremethylester;

4-[17β-Hydroxy-3-oxo-17α-(1,1,2,2,2-pentafluorethyl)estr-4-en-11β-yl]benzoesäuremethylester;

9,11 α-Dihydro-17β-hydroxy-3-oxo-17α-(1,1,2,2,2-pentafluorethyl)-4'*H*naphth[3',2',1':10,9,11]estr-4-en-6'-carbonsäuremethylester;

17β-Hydroxy-11β-[4-(1-hydroxyethyl)phenyl]-17α-(1,1,2,2,2-pentafluorethyl)estra-4,9-dien-3-on;

17β-Hydroxy-11β-[4-(1-hydroxyethyl)phenyl]-17α-(1,1,2,2,2-pentafluorethyl)estr-4-en-3-on;

9,11α-Dihydro-17β-hydroxy-6'-(1-hydroxyethyl)-17α-(1,1,2,2,2-pentafluorethyl)-4'*H*naphth[3',2',1':10,9,11]estr-4-en-3-on.

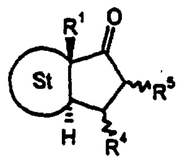

II

Die Etablierung der fluorierten Seitenketten in 17α-Position erfolgt in Analogie zu vielfach für andere Seitenketten beschrieben Verfahren durch nukleophile Addition einer Metall-organischen Verbindung der Formel $MC_nF_mH_o$ an ein 17-Keton der allgemeinen Formel II, wobei M für ein Metall in der Bedeutung von z.B. Li, Na, K, Mg-Halogen (Halogen = Cl, Br, I) oder anderer Metalle steht und n, m und o die bereits in der allgemeinen Formel I angegebene Bedeutung haben. Zu bevorzugen ist die Addition von Grignardreagenzien ($C_nF_mH_o$Mg-Halogen) bzw. der Lithium-organischen Verbindungen des Typs $LiC_nF_mH_o$. Für die Einführung perfluorierter Seitenketten ist die Generierung der Lithium-organischen Reagenzien ausgehend von den entsprechenden Iodiden mittels Methyllithium/Lithiumbromid-Komplex ( J. Org. Chem. **1987**, *52*,2481 und Tetrahedron Lett. **1985**, 26, 5243) besonders gut geeignet.

Die in der allgemeinen Formel II genannten Substituenten $R^1$, $R^4$, $R^5$ und St haben die bereits in der allgemeinen Formel I angegebenen Bedeutungen, wobei in St vorhandene funktionelle Gruppen gegebenenfalls nach dem Fachmann bekannten Verfahren geschützt werden können. Besonders Carbonylgruppen, wie z.B. die 3-Ketogruppierung werden in den meisten Fällen in geeigneter Weise, z.B. durch Bildung eines entsprechenden Ketals oder Reduktion zu einer Hydroxygruppe und gegebenenfalls Überführung dieser Hydroxygruppe in einen Ether oder Ester, geschützt.

Als Ketalschutzgruppe sind beispielsweise die Ethylendioxy- oder die 2,2-Dimethylpropylen-1,3-dioxygruppe zu nennen. Auch andere gängige Ketoschutzgruppen kommen in Betracht. Im Falle einer geschützten Hydroxygruppe kann diese beispielsweise in Form des Methoxymethyl-, Methoxyethyl-, Tetrahydroxypyranyl- oder Silylethers geschützt werden. Durch Abspaltung der Schutzgruppe und Oxidation der freien Hydroxygruppe gelangt man zur Ketogruppe.

Auf einer geeigneten Stufe nach erfolgter Addition der 17α-Seitenkette werden dann die Schutzgruppen in bekannter Weise entfernt und gegebenenfalls eine Hydroxygruppe zur korrespondierenden Ketogruppe oxidiert.

Die Addition der 17α-Seitenkette kann aber auch in Gegenwart weiterer freier Carbonylgruppen, z.B. auch der 3-Ketogruppe selektiv erfolgen.

Die zur Herstellung der Verbindungen der allgemeinen Formel I dienenden Ausgangsmaterialien der allgemeinen Formel II sind in einer ganzen Reihe von Patenten, Patentanmeldungen und Veröffentlichungen beschrieben:

EP-A 0 057 115, EP-A 0 129 499, EP-A 0 259 2489, EP-A 0 186 834, EP-A 0 447 014, EP-A 0 116 974, EP-A 0 190 759, EP-A 0 147 361, EP-A 192 598, EP-A 0 283 428, EP-A 0 404 283, WO-A 89/00578, WO-A 91/18917, WO-A 91/18918, WO-A 92/11277, WO-A 92/11278, WO-A 93/23020, Steroids 44 (1984), 349 sowie weiteren, dem auf diesem Gebiet tätigen Fachmann bekannten, einschlägigen Literaturstellen.

In den genannten Schutzrechten ist auch die Einführung der zu den hier beanspruchten Reste $R^4$, $R^5$, $R^6$, $R^7$ und $R^8$ dort analog vorkommenden Reste beschrieben.

Generell kann die Addition der Seitenkette auf jeder Synthesezwischenstufe mit einer freien 17-Ketogruppe erfolgen.

Wird die Einführung der fluorierten 17α-Alkylseitenkette auf einer frühen Synthesezwischenstufe durchgeführt, so kann die Etablierung der weiteren in St genannten Reste $R^6$, $R^7$ und $R^8$ in Gegenwart dieser 17α-Seitenkette nach bekannten Verfahren durchgeführt werden, wie sie unter anderem in den oben genannten Patenten, Patentanmeldungen und Publikationen beschrieben wurden.

Die neuen Verbindungen der allgemeinen Formel I sind wertvolle pharmazeutische Wirkstoffe. Sie zeichnen sich durch eine sehr starke antigestagene Wirksamkeit aus. Es handelt sich um kompetitive Progesteronantagonisten, da sie das Progesteron von seinem Rezeptor verdrängen. Gleichzeitig sind andere endokrine Nebenwirkungen, wie z.B. androgene, estrogene oder antiglucocorticoide Aktivität wenn überhaupt nur in sehr geringem Ausmaß verhanden. Die Verbindungen können daher für medizinische Zwecke genutzt werden.

Verbindungen mit antigestagener Wirksamkeit (kompetitive Progesteronantagonisten) sind erstmals 1982 bekannt geworden (RU 486 = EP-A 0 057 115) und seither zahlreich beschrieben worden, u.a. in den bereits genannten Patent- und Literaturfundstellen.

Unter den bisher offenbarten Verbindungen finden sich keine solchen mit einer mehrfach fluorierten, mindestens 2 Kohlenstoffatome enthaltenden 17α-Alkylseitenkette. Lediglich in der WO83/03099 ist gesagt, daß die dort offenbarten 3-Keto-$\Delta^{4,9}$-19-nor-steroide eine 17α-Alkylseitenkette tragen können, die gegebenenfalls mit einem Halogenatom substituiert sein kann. Fluor als Halogen ist nicht genannt.

In Chemical Abstracts 1995, vol 123, abstr. No. 286388 und Journal of Fluorine Chemistry 1994, Bd. 69, 1-3 wird ein verbessertes Verfahren zur Einführung einer Trifluormethylgruppe in Position 17α eines Estra-4,9-diens beschrieben. Als Beispiel wird die Herstellung von 11β-(4-Dimethylaminophenyl)-17β-hydroxy-17α-trifluormethyl-estra-4,9-dien-3-on angeführt. Die Substanz wird als wirksames Antigestagen charakterisiert. Detaillierte biologische Merkmale sind nicht beschrieben.

Konkrete Beispiele mit einer mindestens 2 Kohlenstoffe aufweisenden 17α-Alkylkette gibt es bisher überhaupt nicht.

Wirkstoffe dieser Art mit starker antigestagener Aktivität sind zur Auslösung von Aborten geeignet, da sie das zur Aufrechterhaltung der Schwangerschaft erforderliche Progesteron vom Rezeptor verdrängen. Sie sind deshalb wertvoll und interessant im Hinblick auf ihre Verwendung zur postcoitalen Fertilitätskontrolle.

Die erfindungsgemäßen Verbindungen der allgemeinen Formel I sind auch zur Herstellung von Präparaten für die Empfängnisverhütung für die Frau geeignet (WO-A 93/23020, WO-A 93/21927).

Sie können außerdem gegen hormonelle Unregelmäßigkeiten, zur Menstruationsauslösung und zur Geburtseinleitung eingesetzt werden. Weitere Indikationsgebiete im Bereich der Gynäkologie sind die Hormonersatz-Therapie (WO-A 94/18983), die Behandlung von den mit einer Dysmenorrhoe einhergehenden Beschwerden und der Endometriose (EP-A 0 266 303) sowie von Myomen.

Die erfindungsgemäßen Verbindungen üben in Progesteronrezeptor-positiven Nager- und humanen Brustkrebsmodellen eine starke Antitumor-Aktivität aus.

Antiproliferative Wirkung wurde *in vitro* an der humanen T47D-Bustkrebszellinie beobachtet. *In vivo* wurden tumorhemmende Effekte am MXT-Mammatumor der Maus und in den mit NMU (N-Nitrosomethylhamstoff) oder DMBA (Dimethylbenzanthrazen) chemisch induzierten Mammacarcinom-Modellen der Ratte nachgewiesen.

Die erfindungsgemäßen Verbindungen sind daher bestens für die Behandlung von hormonabhängigen Carcinomen geeignet, wie beispielsweise des Progesteronrezeptor-positiven Mammacarcinoms.

Die erfindungsgemäßen Verbindungen können in der Therapie hormonabhängiger Carcinome sowohl für die first-line Therapie als auch für die second-line Therapie, insbesondere nach Tamoxifen-failure, zum Einsatz kommen.

Die erfindungsgemäßen, antigestagen wirksamen Verbindungen der allgemeinen Formel I können auch in Kombination mit antiestrogen wirksamen Verbindungen zur Herstellung pharmazeutischer Präparate zur Behandlung hormonabhängiger Tumoren (EP-A 0 310 542), zur Geburtseinleitung, zum Schwangerschaftsabbruch sowie zur Behandlung gynäkologischer Störungen (EP-A 0 310 541) und zur weiblichen Kontrazeption (WO 96/19997) verwendet werden.

Bei der Behandlung hormonabhängiger Tumore können das Antigestagen und das Antiestrogen zur gleichzeitigen oder auch zur sequentiellen Verabreichung vorgesehen sein. Bei der sequentiellen Verabreichung wird vorzugsweise zuerst das Antiestrogen und anschließend das Antigestagen verabreicht.

Zur Kombination mit den erfindungsgemäßen Antigestagenen kommen dabei beispielsweise die folgenden Antiestrogene in Betracht: Tamoxifen, ICI 182.780 (= 7α-[9-(4,4,5,5,5-Pentafluorpentylsulfinyl)nonyl]estra-1,3,5(10)-trien-3,17β-diol), die in der PCI/EP97/04517 beschriebenen antiestrogenen Verbindungen und Aromataseinhibitoren, beispielsweise Fadrozol, Formestan, Letrozol, Anastrozol oder Atamestan.

Als Antigestagene wurden untersucht:

A: 11β-(4-Acetylphenyl)-17β-hydroxy-17α-(1,1,2,2,2-pentafluorethyl)estr-4-en-3-on (Beispiel 1)
B: 11 β-(4-Acetylphenyl)-17β-hydroxy-17α-(1,1,2,2,2-pentafluorethyl)estra-4,9-dien-3-on (Beispiel 3)
C: 6'-Acetyl-9,11 α-dihydro-17β-hydroxy-17α-(1,1,2,2,2-pentafluorethyl)-4'*H*naphth[3',2',1':10,9,11]estr-4-en-3-on (Beispiel 5)
D: 11β-[4-(Dimethylamino)phenyl]-17β-hydroxy-17α-(1-propinyl)estra-4,9-dien-3-on (RU 38 486)
E: 11β-(4-Acetylphenyl)-19,24-dinor-17,23-epoxy-17α-chola-4,9,20-trien-3-on (Org 33 628)

Die nachstehenden Tests wurden alle nach bekannten Methoden an der Ratte durchgeführt.

Abortivtest s.c. und p.o.: siehe beispielsweise EP-A 0 283 428

Androgentest p.o.: Stimulation des Prostatagewichts mit der Testverbindung, Vehikel: s.c. Benzylbenzoat/Rizinusöl (1+4); p.o. NaCl-Myrj; Referenzverbindung Testosteronpropionat. Bis zu einer Dosis von 10 mg Testverbindung/Tier/Tag wird praktisch keine Stimulation des Prostatagewichts beobachtet

Uteruswachstumstest p.o. auf estrogene Wirkung: Stimulation des Uterusgewichts mit der Testverbindung, Vehikel: s.c. Benzylbenzoat/Rizinusöl (1+4); p.o. NaCl-Myrj; 3tägige Behandlung ovarektomierter Tiere; Parameter: Uterusgewicht und Endometriumsepithelhöhe; Vaginalabstrich negativ; Referenzverbindung: Estradiol 0,1µg

Antithymolysetest p.o. auf antiglucocorticoide Wirkung: siehe beispielsweise EP-A 0 283 428

| | Verbindung A | Verbindung B | Verbindung C | Verbindung D | Verbindung E |
|---|---|---|---|---|---|
| Abortivtest Ratte s.c. Dosis [mg/Tier/Tag] (n Abort/n Gesamt) | 0,3 (4/4)<br>0,1 (4/4)<br>0,03 (4/4) | 0,3 (4/4)<br>0,1 (4/4)<br>0,03 (4/4) | 0,3 (4/4)<br>0,1 (4/4)<br>0,03 (4/4) | 3 (4/4)<br>1 (3/4)<br>0,3 (0/6) | 0,3 (4/4)<br>0,1 (3/4)<br>0,03 (0/4) |
| Abortivtest Ratte p.o. Dosis [mg/Tier/Tag] (n Abort/n Gesamt) | 0,3 (4/4)<br>0,1(4/4)<br>0,03 (4/4) | 0,3 (4/4)<br>0,1 (4/4)<br>0,03 (4/4) | 0,3 (4/4)<br>0,1 (4/4)<br>0,03 (4/4)<br>0,01 (4/4)<br>0,003 (4/4) | 3 (4/4)<br>1 (2/4)<br>0,3 (0/4) | 0,3 (4/4)<br>0,1 (4/4)<br>0,03 (0/4) |
| Androgentest Ratte p.o. Dosis [mg/Tier/Tag] (% Prostatastimulation) | 3 (3,3)<br>1(4,8) | 3 (0)<br>1 (2,1) | n. b. | 10 (7,2)<br>3 (2,9)<br>1 (1,6) | 10 (4,4)<br>3 (5,6)<br>1 (4,0) |
| Uteruswachstumtest Ratte p. o. Dosis [mg/Tier/Tag] (% Uterusgewichtstimulation) | 10 (3,7) | 10 (2,6) | 10 (4,4) | 10 (6,4) | 10 (2,4)<br>3 (1,3) |
| Antithymolysetest Ratte p.o. Dosis [mg/Tier/Tag] (% Aufhebung der Dexamethason-induzierten Thymus-Suppression) | 10(11,5)<br>3 (7,4)<br>1 (6,0) | 10 (18,2)<br>3 (21,6)<br>1 (1,2) | 22 (18,8)<br>6,7 (31,6)<br>2,2 (7,2) | 10 (76)<br>3 (79)<br>1 (19) | 10 (44,7)<br>3 (19,1)<br>1 (4,3) |
| n. b.: nicht bestimmt | | | | | |

Die Erfindung betrifft somit auch Arzneimittel auf Basis der pharmazeutisch verträglichen, d.h. in den verwendeten Dosen nicht toxischen Verbindungen der allgemeinen Formel I, gegebenenfalls in Verbindung mit einem Antiestrogen, zusammen mit den üblichen Hilfs- und Trägerstoffen.

Schließlich betrifft die vorliegende Erfindung auch die Verwendung der Verbindungen der allgemeinen Formel I, gegebenenfalls zusammen mit einem Antiestrogen, zur Herstellung von Arzneimitteln.

Die erfindungsgemäßen Verbindungen können nach an sich bekannten Methoden der Galenik zu pharmazeutischen Präparaten für die enterale, percutane, parenterale oder lokale Applikation verarbeitet werden. Sie können in Form von Tabletten, Dragees, Gelkapseln, Granulaten, Suppositorien, Implantaten, injizierbaren sterilen wäßrigen oder öligen Lösungen, Suspensionen oder Emulsionen, Salben, Cremes und Gelen oder mittels intravaginaler (z.B. Vaginalringe) oder intrauteriner Syteme (Pessare, Spiralen) verabreicht werden.

Der oder die Wirkstoffe können dabei mit den in der Galenik üblichen Hilfsstoffen wie z.B. Gummiarabikum, Talk, Stärke, Mannit, Methylcellulose, Laktose, Tensiden wie Tweens oder Myrj, Magnesiumstearat, wäßrigen oder nicht wäßrigen Trägern, Paraffinderivaten, Netz-, Dispergier-, Emulgier-, Konservierungsmitteln und Aromastoffen zur Geschmackskorrektur (z.B. etherischen Ölen) gemischt werden.

Eine Dosiseinheit enthält etwa 0,1-100 mg Wirkstoff(e). Die Dosierung der erfindungsgemäßen Verbindungen liegt beim Menschen bei etwa 0,1-400 mg pro Tag.

Die nachfolgenden Beispiele dienen der näheren Erläuterung der vorliegenden Erfindung:

**Beispiel 1**

11β-(4-Acetylphenyl)-17β-hydroxy-17α-(1,1,2,2,2-pentafluorethyl)estr-4-en-3-on

1a) 3,3;17,17-Bis[1,2-ethandiylbis(oxy)]-11β-[4-[[(1,1,2,2,3,3,4,4,4-nonafluorbutyl)sulfonyl]oxy]phenyl]estr-5-en

Zu einer Lösung von 6 g 4-[3,3;17,17-Bis[1,2-ethandiylbis(oxy)]estr-5-en-11β-yl]phenol, dessen Herstellung in WO 91/18917 und WO 91/18918 beschrieben ist, in 100 ml absolutem Tetrahydrofuran werden bei 0°C 9,2 ml einer 1,6 molaren Lösung von Butyllithium in Hexan addiert. Man läßt 30 Minuten bei 0°C nachrühren und addiert dann 5 ml 1,1,2,2,3,3,4,4,4-Nonafluor-1-butansulfonylfluorid. Es wird eine Stunde bei 0°C nachgerührt. Anschließend wird das Reaktionsgemisch auf gesättigte wäßrige Natriumhydrogencarbonatlösung gegossen. Man extrahiert mit Ethylacetat, wäscht mit gesättigter wäßriger Natriumchloridlösung, trocknet über Natriumsulfat und engt im Vakuum ein. Säulenchromatographie des Rohprodukts an Kieselgel mit einem Gemisch aus Hexan/Ethylacetat ergibt 8,2 g 1a) als weißen Schaum.
$^1$H-NMR ($CDCl_3$): δ= 7,45 d (J=9 Hz, 2H, aryl); 7,17 d (J=9 Hz, 2H, aryl); 5,55 dbr (J=5 Hz, 1H, H-6); 4,00-3,80 m (8H, ketale); 3,50 ddbr (J=7 Hz + 5 Hz, 1H, H-11); 0,53 s (3H, H-18).

1b) 3,3-[1,2-Ethandiylbis(oxy)]-11β-[4-[[(1,1,2,2,3,3,4,4,4-nonafluorbutyl)sulfonyl]oxy]phenyl]estr-5-en-17-on

8,2 g der unter 1a) beschriebenen Verbindung werden mit 22 g Kieselgel und 2 ml gesättigter wäßriger Oxalsäurelösung in 85 ml Dichlormethan 5 Stunden bei Raumtemperatur gerührt. Anschließend wird über Celite filtriert. Man engt im Vakuum ein und reinigt das Rohprodukt durch Kristallisation aus Diisopropylether. Man erhält 5,3 g 1b) als weiße Kristalle.
$^1$H-NMR ($CDCl_3$): δ= 7,45 d (J=9 Hz, 2H, aryl); 7,19 d (J=9 Hz, 2H, aryl); 5,59 dbr (J=5 Hz, 1H, H-6); 4,00-3,88 m (4H, ketal); 3,52 ddbr (J=7 Hz + 5 Hz, 1H, H-11); 0,55 s (3H, H-18).

1c) 3,3-[1,2-Ethandiylbis(oxy)]-11β-[4-[[(1,1,2,2,3,3,4,4,4-nonafluorbutyl)sulfonyl]oxy]phenyl]-17α-(1,1,2,2,2-pentafluorethyl)estr-5-en-17β-ol

1 ml kondensiertes Pentafluorethyliodid wird mit einer Lösung von 691 mg 1b) in 10 ml absolutem Diethylether bei -78°C versetzt. Man addiert bei dieser Temperatur 4,77 ml einer 1,5 molaren Lösung von Methyllithium-Lithiumbromid-Komplex in Diethylether. Anschließend wird eine Stunde bei -78°C nachgerührt. Dann wird das Reaktionsgemisch auf gesättigte wäßrige Natriumhydrogencarbonatlösung gegossen. Man extrahiert mit Ethylacetat, wäscht mit gesättigter wäßriger Natriumchloridlösung, trocknet über Natriumsulfat und engt im Vakuum ein. Chromatographie des erhaltenen Rohprodukts an Kieselgel mit einem Gemisch aus Hexan/Ethylacetat ergibt 719 mg 1c) als weißen Schaum.
$^1$H-NMR ($CDCl_3$): δ= 7,45 d (J=9 Hz, 2H, aryl); 7,19 d (J=9 Hz, 2H, aryl); 5,54 dbr (J=5 Hz, 1H, H-6); 3,88-4,00 m (4H, ketal); 3,53 ddbr (J=7 Hz + 5 Hz, 1H, H-11); 0,60 s (3H, H-18).

1d) 11β-(4-Acetylphenyl)-3,3-[1,2-ethandiylbis(oxy)]-17α-(1,1,2,2,2-pentafluorethyl)estr-5-en-17β-ol

Eine Lösung aus 719 mg 1c), 0,45 ml (1-Ethoxyethenyl)tributylstannan, 41 mg Tetrakis(triphenylphosphin) palladium(0), 263 mg Lithiumchlorid und 0,1 ml Pyridin in 12ml Dioxan wird 1,5 Stunden unter Rückflüß gekocht. Anschließend wird das Reaktionsgemisch auf Wasser gegossen. Man extrahiert mit Ethylacetat und addiert zur organischen Phase gesättigte wäßrige Ammoniumchloridlösung sowie 3 ml gesättigte wäßrige Oxalsäurelösung. Es wird 30 Minuten bei Raumtemperatur nachgerührt. Anschließend wird die organische Phase abgetrennt und mit gesättigter wäßriger Natriumhydrogencarbonat- sowie mit gesättigter wäßriger Natriumchloridlösung gewaschen. Man trocknet über Natriumsulfat und engt im Vakuum ein. Säulenchromatographie des erhaltenen Rohprodukts an Kieselgel mit einem Gemisch aus Hexan/Ethylacetat ergibt 440 mg 1d).
$^1$H-NMR ($CDCl_3$): δ= 7,88 d (J=9 Hz, 2H, aryl); 7,47 d (J=9 Hz, 2H, aryl); 5,55 dbr (J=5 Hz, 1H, H-6); 3,88-4,00 m (4H, ketal); 3,55 ddbr (J=7 Hz + 5 Hz, 1H, H-11); 2,61 s (3H, acetyl); 0,62 s (3H, H-18).

1e) 11β-(4-Acetylphenyl)-17β-hydroxy-17α-(1,1,2,2,2-pentafluorethyl)estr-4-en-3-on

440 mg 1d) werden in 10 ml Aceton gelöst. Man addiert 1 ml 4 N wäßrige Salzsäure und rührt 1,5 Stunden bei Raumtemperatur nach. Anschließend wird das Reaktionsgemisch auf gesättigte Natriumhydrogencarbonatlösung gegossen. Man extrahiert mit Dichlormethan, wäscht die organische Phase mit gesättigter wäßriger Natriumchloridlösung, trocknet über Natriumsulfat und engt im Vakuum ein. Säulenchromatographie des erhaltenen Rohprodukts an Kieselgel mit einem Gemisch aus Hexan/Ethylacetat ergibt 311 mg 1e) als weißen Schaum.

$^1$H-NMR ($CDCl_3$): δ= 7,89 d (J=9 Hz, 2H, aryl); 7,53 d (J=9 Hz, 2H, aryl); 5,89 sbr (1H, H-4); 3,50 ddbr (J=7 Hz + 5 Hz, 1H, H-11); 2,84 m (1H, H-10); 2,60 s (3H, acetyl); 0,70 s (3H, H-18).

**Beispiel 2**

11β-(4-Acetylphenyl)-17β-hydroxy-17α-(1,1,2,2,3,3,4,4,4-nonafluorbutyl)estr-4-en-3-on

2a) 3,3-[1,2-Ethandiylbis(oxy)]-17α-(1,1,2,2,3,3,4,4,4-nonafluorbutyl)-11β-[4-[[(1,1,2,2,3,3,4,4,4-nonafluorbutyl)sulfonyl]oxy]phenyl]estr-5-en-17β-ol

Analog zu Beispiel 1c) werden 691 mg 1b) in 15 ml absolutem Diethylether mit dem aus 0,52 ml 1-Iod-1,1,2,2,3,3,4,4,4-nonafluorbutan und 1,67 ml einer 1,5 molaren Lösung von Methyllithium-Lithiumbromid-Komplex in Diethylether gebildeten Reagenz umgesetzt. Man erhält nach Chromatographie an Kieselgel mit einem Gemisch aus Hexan/Ethylacetat 838 mg 2a) als weißen Schaum.
$^1$H-NMR ($CDCl_3$): δ= 7,44 d (J=9 Hz, 2H, aryl); 7,18 d (J=9 Hz, 2H, aryl); 5,55 dbr (J=5 Hz, 1H, H-6); 3,88-4,00 m (4H, ketal); 3,53 ddbr (J=7 Hz + 5 Hz, 1H, H-11); 0,61 s (3H, H-18).

2b) 11β-(4-Acetylphenyl)-3,3-[1,2-Ethandiylbis(oxy)]-17α-(1,1,2,2,3,3,4,4,4-nonafluorbutyl)estr-5-en-17β-ol

Analog zu Beispiel 1d) werden 838 mg 2a) mit 0,46 ml (1-Ethoxyethenyl)tributylstannan, 43 mg Tetrakis(triphenylphosphin)palladium(0), 272 mg Lithiumchlorid und 0,1 ml Pyridin in 12 ml Dioxan umsetzt. Man erhält nach Aufarbeitung sowie Behandlung mit gesättigter wäßriger Ammoniumchloridlösung sowie gesättigter wäßriger Oxalsäurelösung und Säulenchromatographie an Kieselgel mit einem Gemisch aus Hexan/Ethylacetat 505 mg 2b) als weißen Schaum.
$^1$H-NMR ($CDCl_3$): δ= 7,85 d (J=9 Hz, 2H, aryl); 7,46 d (J=9 Hz, 2H, aryl); 5,55 dbr (J=5 Hz, 1H, H-6); 3,88-4,00 m (4H, ketal); 3,55 ddbr (J=7 Hz + 5 Hz, 1H, H-11); 2,61 s (3H, acetyl); 0,63 s (3H, H-18).

2c) 11β-(4-Acetylphenyl)-17β-hydroxy-17α-(1,1,2,2,3,3,4,4,4-nonafluorbutyl)estr-4-en-3-on

Analog zu Beispeil 1e) werden 505 mg 2b) mit 4 N Salzsäure in Aceton umgesetzt. Man erhält nach Säulenchromatographie an Kieselgel mit einem Gemisch aus Hexan/Ethylacetat 372 mg 2c) als weißen Schaum.
$^1$H-NMR ($CDCl_3$): δ= 7,89 d (J=9 Hz, 2H, aryl); 7,55 d (J=9 Hz, 2H, aryl); 5,88 sbr (1H, H-4); 3,51 ddbr (J=7 Hz + 5 Hz, 1H, H-11); 2,85 m (1H, H-10); 2,60 s (3H, acetyl); 0,70 s (3H, H-18).

**Beispiel 3**

11β-(4-Acetylphenyl)-17β-hydroxy-17α-(1,1,2,2,2-pentafluorethyl)estra-4,9-dien-3-on

3a) 3,3-[2,2-Dimethyl-1,3-propandiylbis(oxy)]-17α-(1,1,2,2,2-pentafluorethyl)-11β-[4-(2,5,5-trimethyl-1,3-dioxolan-2-yl)phenyl]-5α-estr-9-en-5,17β-diol

Analog zu Beispiel 1c) werden 1,08 g 3,3-[2,2-Dimethyl-1,3-propandiylbis(oxy)]-5-hydroxy-11β-[4-(2,5,5-trimethyl-1,3-dioxolan-2-yl)phenyl]-5α-estr-9-en-17-on, dessen Herstellung in EP 0190759, Beispiel 6c) beschrieben ist, in 19 ml absolutem Diethylether mit dem aus 1,9 ml 1-Iod-1,1,2,2,2-pentafluorethan und 8,7 ml einer 1,5 molaren Lösung von Methyllithium-Lithiumbromid-Komplex in Diethylether gebildeten Reagenz umgesetzt. Man erhält nach Säulenchromatographie an Kieselgel mit einem Gemisch aus Hexan/Ethylacetat 644 mg der Titelverbindung als farblosen Schaum.
$^1$H-NMR ($CDCl_3$): δ= 7,29 d (J=9 Hz, 2H, aryl); 7,23 d (J=9 Hz, 2H, aryl); 4,42 s (1H, 5-OH); 4,35 dbr (J=7 Hz, 1H, H-11); 1,52 s (3H, arylketal); 1,26 s (3H, arylketal); 1,04 s (3H, 3-ketal); 0,89 s (3H, 3-ketal); 0,57 s (3H, arylketal); 0,51 s (3H, H-18).

3b) 11β-(4-Acetylphenyl)-17β-hydroxy-17α-(1,1,2,2,2-pentafluorethyl)estra-4,9-dien-3-on

635 mg der unter 3a) beschriebenen Verbindung werden in 9 ml Methanol mit 0,4 ml wäßriger halbkonzentrierter Schwefelsäure zwei Stunden bei Raumtemperatur gerührt. Anschließend wird auf gesättigte wäßrige Natriumhydrogencarbonatlösung gegossen und mit Ethylacetat extrahiert. Man wäscht die organische Phase mit gesättigter wäßriger Natriumchloridlösung, trocknet über Natriumsulfat, filtriert und engt im Vakuum ein. Säulenchromatographie an Kieselgel mit einem Gemisch aus Hexan/Ethylacetat ergibt 428 mg der Titelverbindung als farblosen Schaum.

Fp.: 260,4°C (Diisopropylether), $[\alpha]_D^{22}$ = +181,3° ($CHCl_3$, c = 0,535)

**Beispiel 4**

11β-(4-Acetylphenyl)-17β-hydroxy-17α-(1,1,2,2,2-pentafluorethyl)estra-4,9,15-trien-3-on

4a) 3,3-[2,2-Dimethyl-1,3-propandiylbis(oxy)]-17α-(1,1,2,2,2-pentafluorethyl)-11β-[4-(2,5,5-trimethyl-1,3-dioxolan-2-yl)phenyl]-5α-estra-9,15-dien-5,17β-diol

Analog zu Beispiel 1c) werden 1,15 g 3,3-[2,2-Dimethyl-1,3-propandiylbis(oxy)]-5-hydroxy-11β-[4-(2,5,5-trimethyl-1,3-dioxolan-2-yl)phenyl]-5α-estra-9,15-dien-17-on, dessen Herstellung in WO 89/00578, Beispiel 1b) beschrieben ist, in 20 ml absolutem Diethylether und 10 ml absolutem Tetrahydrofuran mit dem aus 2,0 ml 1-Iod-1,1,2,2,2-pentafluorethan und 9,3 ml einer 1,5 molaren Lösung von Methyllithium-Lithiumbromid-Komplex in Diethylether gebildeten Reagenz umgesetzt. Man erhält nach Säulenchromatographie an Kieselgel mit einem Gemisch aus Hexan/Ethylacetat 1,16 g der Titelverbindung als farblosen Schaum.
$^1$H-NMR ($CDCl_3$): δ= 7,31 d (J=9 Hz, 2H, aryl); 7,24 d (J=9 Hz, 2H, aryl); 6,31 dbr (J=6 Hz, 1H, H-15); 5,58 dddbr (J=6 Hz + 3.5 Hz + 1.5 Hz, 1H, H-16); 4,49 s (1H, 5-OH); 4,40 dbr (J=8 Hz, 1H, H-11); 1,52 s (3H, arylketal); 1,26 s (3H, arylketal); 1,03 s (3H, 3-ketal); 0,89 s (3H, 3-ketal); 0,68 s (3H, H-18); 0,58 s (3H, arylketal).

4b) 11β-(4-Acetylphenyl)-17β-hydroxy-17α-(1,1,2,2,2-pentafluorethyl)estra-4,9,15-trien-3-on

1,15 g der unter 4a) beschriebenen Verbindung werden analog zu dem in 3b) beschriebenen Verfahren in 16,5 ml Methanol mit 0,73 ml wäßriger halbkonzentrierter Schwefelsäure zu 572 mg der Titelverbindung als farblosem Schaum umgesetzt.
Fp.: 213,9°C (Diisopropylether), $[\alpha]_D^{22}$ = +210,5° ($CHCl_3$, c = 0,615)

**Beispiel 5**

9,11α-Dihydro-6'-(4-fluorphenyl)-17β-hydroxy-17α-(1,1,2,2,2-pentafluorethyl)-4'*H*naphth[3',2',1':10,9,11]estr-4-en-3-on

5a) 9,11α-Dihydro-3,3-[2,2-dimethyl-1,3-propandiylbis(oxy)]-6'-[[(1,1,2,2,3,3,4,4,4-nonafluorbutyl)sulfonyl]oxy]-17α-(1,1,2,2,2-pentafluorethyl)-4'*H*-naphth[3',2',1':10,9,11]-5α-estran-5,17β-diol

Die Lösung von ca. 4 ml Pentafluorethyliodid in 20 ml wasserfreiem Ether versetzt man bei -78°C unter einer Atmosphäre aus trockenem Argon mit 13,4 ml einer 1,7 molaren Lösung von tert.-Butyllithium in Hexan und rührt 30 Minuten. Anschließend tropft man die Lösung von 2,0 g (2,62 mmol) 9,11α-Dihydro-3,3-[2,2-dimethyl-1,3-propandiylbis(oxy)]-5-hydroxy-6'-[[(1,1,2,2,3,3,4,4,4-nonafluorbutyl)sulfonyl]oxy]-4'*H*-naphth[3',2',1':10,9,11]-5α-estran-17-on, das man in Analogie zu dem in DE 4216003 (Bsp. 1b) beschriebenen Verfahren hergestellt hat, in 60 ml wasserfreiem Toluol zu und läßt innerhalb von 2 Stunden auf-10°C erwärmen. Man gießt in eine gesättigte wäßrige Natriumhydrogencarbonatlösung, extrahiert mehrfach mit Ethylacetat, wäscht die vereinigten organischen Extrakte mit einer gesättigten wäßrigen Natriumchloridlösung und trocknet über Natriumsulfat. Den nach Filtration und Lösungsmittelabzug erhaltenen Rückstand reinigt man durch Chromatographie an ca. 300 ml feinem Kieselgel mit einem Gradientensystem aus Hexan und Ethylacetat. Man isoliert 1,40 g (1,59 mmol, 61%) der Titelverbindung als farblosen Feststoff sowie 730 mg (0,96 mmol, 36%) Ausgangsmaterial.
$^1$H-NMR ($CDCl_3$): δ=0,41 (3H), 0,93 (3H), 1,00 (3H), 1,20-1,36 (2H), 1,42-1,81 (11H), 1,93 (2H), 2,07-2,28 (3H), 2,31-2,48 (1H), 2,61-2,77 (2H), 3,15 (1H), 3,21 (1H), 3,45-3,65 (4H), 4,48 (1H), 6,98 (1H), 7,04 (1H), 7,47 (1H) ppm.

5b) 9,11α-Dihydro-3,3-[2,2-dimethyl-1,3-propandiylbis(oxy)]-6'-(4-fluorphenyl)-17α-(1,1,2,2,2-pentafluorethyl)-4'*H*-naphth[3',2',1':10,9,11]-5α-estran-5,17β-diol

Die Lösung von 400 mg (453 μmol) der nach Beispiel 5a dargestellten Verbindung in einem Gemisch aus 7 ml wasserfreiem Toluol und 3 ml wasserfreiem Ethanol versetzt man nacheinander mit 43 mg Lithiumchlorid, 0,66 ml einer 2 M Natriumcarbonatlösung, 82 mg (4-Fluorphenyl)boronsäure, 50 mg Tetrakis(triphenylphosphin)palladium(0) und erhitzt unter einer Atmosphäre aus Argon 1,5 Stunden auf 95°C. Das Reaktionsgemisch wird mit Wasser verdünnt, mit Ethylacetat extrahiert, die vereinigten organischen Extrakte mit einer gesättigten wäßrigen Natriumchloridlösung gewaschen und über Natriumsulfat getrocknet. Den nach Filtration und Lösungsmittelabzug erhaltenen Rückstand reinigt man durch Chromatographie an ca. 150 ml feinem Kieselgel mit einem Gradientensystem aus Hexan und Ethylacetat. Man isoliert 264 mg (389 µmol, 86%) der Titelverbindung als farblosen Feststoff.
$^1$H-NMR ($CDCl_3$): δ=0,49 (3H), 0,93 (3H), 0,99 (3H), 1,21-2,28 (18H), 2,30-2,47 (1H), 2,76 (2H), 3,17 (1H), 3,26 (1H), 3,47-3,66 (4H), 4,48 (1H), 7,11 (2H), 7,23 (1H), 7,33 (1H), 7,45 (1H), 7,54 (2H) ppm.

5c) 9,11α-Dihydro-6'-(4-fluorphenyl)-17β-hydroxy-17α-(1,1,2,2,2-pentafluorethyl)-4'*H*naphth[3',2',1':10,9,11]estr-4-en-3-on

Die Lösung von 260 mg (383 µmol) der nach Beispiel 5b) dargestellten Verbindung in 13 ml Aceton versetzt man mit 700 µl wäßriger 4 N Salzsäure und erhitzt 4 Stunden auf 50°C. Man gießt in eine gesättigte wäßrige Natriumhydrogencarbonatlösung, extrahiert mehrfach mit Dichlormethan, wäscht die vereinigten organischen Extrakte mit einer gesättigten wäßrigen Natriumchloridlösung und trocknet über Natriumsulfat. Den nach Filtration und Lösungsmittelabzug erhaltenen Rückstand reinigt man durch Chromatographie an ca. 100 ml feinem Kieselgel mit einem Gradientensystem aus Hexan und Ethylacetat. Man isoliert 206 mg (359 µmol, 94%) der Titelverbindung als kristallinen Feststoff.
$^1$H-NMR ($CDCl_3$): δ= 0,55 (3H), 1,22 (1H), 1,33-1,50 (2H), 1,54-1,89 (5H), 1,92-2,54 (8H), 2,66 (1H), 2,81 (1H), 2,87 (1H), 3,31 (1H), 3,43 (1H), 5,90 (1H), 7,12 (2H), 7,27 (1H), 7,37 (1H), 7,45-7,60 (3H) ppm.

**Beispiel 6**

6'-Acetyl-9,11α-dihydro-17β-hydroxy-17α-(1,1,2,2,2-pentafluorethyl)-4'*H*naphth[3',2',1':10,9,11]estr-4-en-3-on

Die Lösung von 600 mg (680 µmol) der nach Beispiel 5a) dargestellten Verbindung in 7 ml wasserfreiem *N,N*-Dimethylformamid versetzt man unter einer Atmosphäre aus trockenem Argon nacheinander mit 69 mg Lithiumchlorid, 381 µl (1-Ethoxyethenyl)tributylstannan, 25 mg Tetrakis(triphenylphosphin)palladium(0) und erwärmt 1,5 Stunden auf 110°C. Nach dem Erkalten versetzt man mit 10 ml Aceton, 1,5 ml wäßriger 4 N Salzsäure, läßt 2 Stunden bei 23°C reagieren und erhitzt anschließend noch 3 Stunden auf 50°C. Man gießt in eine gesättigte wäßrige Natriumhydrogencarbonatlösung, extrahiert mehrfach mit Dichlormethan, wäscht die vereinigten organischen Extrakte mit einer gesättigten wäßrigen Natriumchloridlösung und trocknet über Natriumsulfat. Aus dem nach Filtration und Lösungsmittelabzug erhaltenen Rückstand erhält man durch Kristallisation aus Dichlormethan und Aceton 206 mg noch verunreinigter Titelverbindung, die man an 10 analytischen Dünnschichtplatten weiter aufreinigt. Als Laufmittel dient ein Gemisch aus Hexan und Ethylacetat, als Elutionsmittel ein Gemisch aus Dichlormethan und Methanol. Isoliert werden 160 mg (306 µmol, 45%) der Titelverbindung als farbloser Feststoff.
$^1$H-NMR($CDCl_3$): δ= 0,47 (3H), 1,21 (1H), 1,31-1,51 (2H), 1,53-1,85 (5H), 1,98 (2H), 2,12-2,52 (6H), 2,54 (3H), 2,64 (1H), 2,82 (1H), 2,88 (1H), 3,31 (1H), 3,42 (1H), 5,91 (1H), 7,54 (1H), 7,71 (1H), 7,77 (1H) ppm.

**Beispiel 7**

4-[9,11α-dihydro-17β-hydroxy-3-oxo-17α-(1,1,2,2,2-pentafluorethyl)-4'*H*naphth[3',2',1':10,9,11]estr-4-en-6'-yl]benzonitril

7a) 4-[9,11α-dihydro-5,17β-hydroxy-3,3-[2,2-dimethyl-1,3-propandiylbis(oxy)]-17α-(1,1,2,2,2-pentafluorethyl)-4'*H*-naphth[3',2',1':10,9,11]-5α-estran-6'-yl]benzonitril

400 mg (453 µmol) der nach Beispiel 5b) dargestellten Verbindung setzt man in Analogie zu Beispiel 5b) unter Verwendung von 4-(5,5-Dimethyl-1,3,2-dioxaborinan-2-yl)benzonitril um und isoliert nach Aufarbeitung und Reinigung 301 mg (439 µmol, 97%) der Titelverbindung als kristallinen Feststoff.

7b) 4-[9,11α-dihydro-17ß-hydroxy-3-oxo-17α-(1,1,2,2,2-pentafluorethyl)-4'*H*naphth[3',2',1':10,9,11]estr-4-en-6'-yl] benzonitril

296 mg (431 µmol) der nach Beispiel 7a) dargestellten Verbindung setzt man in Analogie zu Beispiel 5c) um und isoliert nach Aufarbeitung und Reinigung 228 mg (392 µmol, 91%) der Titelverbindung als kristallinen Feststoff.
$^1$H-NMR ($CDCl_3$): δ= 0,53 (3H), 1,22 (1H), 1,35-1,51 (2H), 1,55-1,88 (5H), 1,92-2,14 (3H), 2,14-2,53 (5H), 2,65 (1H), 2,81 (1H), 2,88 (1H), 3,32 (1H), 3,45 (1H), 5,91 (1H), 7,32 (1H), 7,42 (1H), 7,55 (1H), 7,70 (4H) ppm.

**Beispiel 8**

17β-Hydroxy-11β-(4-hydroxyphenyl)-17α-(1,1,2,2,2-pentafluorethyl)estra-4,9-dien-3-on

8a) 3,3-[2,2-Dimethyl-1,3-propandiylbis(oxy)]-5-hydroxy-11β-[4-(phenylmethoxy)phenyl]-5α-estr-9-en-17-on

1,17 g Magnesiumspäne werden unter Schutzgas in 4 ml absolutem Tetrahydrofuran vorgelegt und mit einem Tropfen 1,2-Dibromethan versetzt. Nach Eintreten der Reaktion wird eine Lösung von 12,7 g 1-Brom-4-(phenylmethoxy)benzol (Herstellung siehe *J. Amer. Chem. Soc.* **42**, 657 (1920)) in 80 ml absolutem Tetrahydrofuran langsam zugetropft. Das Reaktionsgemisch wird zum Rückfluß erhitzt bis das Magnesium vollständig umgesetzt ist. Anschließend wird auf 0°C abgekühlt und mit 2,39 g Kupfer(I)chlorid versetzt. Eine Lösung von 3 g 3,3-[2,2-Dimethyl-1,3-propandiylbis(oxy)]-5,10-epoxy-5α,10α-estr-9(11)-en-17-on (Herstellung siehe *Tetrahedron Lett.* **26,** 2069-2072 (1985)) in 80 ml absolutem Tetrahydrofuran wird langsam zugetropft. Das Reaktionsgemisch wird über Nacht bei Raumtemperatur gerührt und dann auf gesättigte wäßrige Ammoniumchloridlösung gegossen. Man extrahiert die wäßrige Phase mit Ethylacetat, vereinigt die organischen Phasen, wäscht sie mit gesättigter wäßriger Natriumchloridlösung und trocknet sie über Natriumsulfat. Man filtriert und engt im Vakuum ein. Säulenchromatographie an Kieselgel mit einem Gemisch aus Hexan/Ethylacetat ergibt 3,7 g der Titelverbindung als farblosen Schaum.
$^1$H-NMR ($CDCl_3$): δ= 7,50-7,27 m (5H, benzyl); 7,13 d (J=9 Hz, 2H, aryl); 6,88 d (J=9 Hz, 2H, aryl); 5,02 s (2H, benzyl); 4,45 s (1H, 5-OH); 4,27 dbr (J=6,5 Hz, 1H, H-11); 1,06 s (3H, 3-ketal); 0,87 s (3H, 3-ketal); 0,50 s (3H, H-18).

8b) 3,3-[2,2-Dimethyl-1,3-propandiylbis(oxy)]-17α-(1,1,2,2,2-pentafluorethyl)-11β-[4-(phenylmethoxy)phenyl]-5α-estr-9-en-5,17β-diol

Analog zu Beispiel 1c) werden 1,35 g der unter 8a) beschriebenen Verbindung in 48 ml absolutem Toluol mit dem aus 1,18 g 1-Iod-1,1,2,2,2-pentafluorethan und 2,4 ml einer 1,5 molaren Lösung von Methyllithium-Lithiumbromid-Komplex in Diethylether gebildeten Reagenz umgesetzt. Man erhält nach Säulenchromatographie an Kieselgel mit einem Gemisch aus Hexan/Ethylacetat 730 mg der Titelverbindung als farblosen Schaum.
$^1$H-NMR ($CDCl_3$): δ=7,50-7,30 m (5H, benzyl); 7,12 d (J=9 Hz, 2H, aryl); 6,88 d (J=9 Hz, 2H, aryl); 5,02 s (2H, benzyl); 4,45 s (1H, 5-OH); 4,29 dbr (J=6 Hz, 1H, H-11); 1,06 s (3H, 3-ketal); 0,87 s (3H, 3-ketal); 0,56 s (3H, H-18).

8c) 3,3-[2,2-Dimethyl-1,3-propandiylbis(oxy)]-17α-(1,1,2,2,2-pentafluorethyl)-11β-(4-hydroxyphenyl)-5α-estr-9-en-5,17β-diol

730 mg der unter 8b) hergestellten Verbindung werden in 11 ml Methanol gelöst und mit 341 mg Ammoniumformiat und 73 mg 10%igem Palladium auf Aktivkohle versetzt. Das Reaktionsgemisch wird zwei Stunden bei Raumtemperatur gerührt, dann über Celite® filtriert. Der Rückstand wird gründlich mit Ethylacetat nachgewaschen. Das Filtrat wird im Vakuum eingeengt. Man erhält 631 mg der Verbindung 8c), die roh weiter umgesetzt wird.

8d) 17β-Hydroxy-11β-(4-hydroxyphenyl)-17α-(1,1,2,2,2-pentafluorethyl)estra-4,9-dien-3-on

631 mg der unter 8c) beschriebenen Verbindung werden analog zu dem in 3b) beschriebenen Verfahren in 11 ml Methanol mit 0,48 ml wäßriger halbkonzentrierter Schwefelsäure zu 428 mg der Titelverbindung als farblosem Schaum umgesetzt.
$^1$H-NMR ($CDCl_3$): δ= 7,00 d (J=9 Hz, 2H, aryl); 6,75 d (J=9 Hz, 2H, aryl); 5,94 sbr (1H, OH); 5,80 s (1H, H-4); 4,38 dbr (J=7 Hz, 1H, H-11); 0,61 s (3H, H-18).

**Beispiel 9**

11β-[4-(Acetyloxy)phenyl]-17β-hydroxy-17α-(1,1,2,2,2-pentafluorethyl)estra-4,9-dien-3-on

300 mg der unter 8d) beschriebenen Verbindung werden in 12 ml Pyridin gelöst und vier Stunden mit 61 µl Essigsäureanhydrid bei Raumtemperatur gerührt. Das Reaktionsgemisch wird auf gesättigte wäßrige Ammoniumchloridlösung gegossen. Man extrahiert die wäßrige Phase mit Ethylacetat, vereinigt die organischen Phasen, wäscht sie mit gesättigter wäßriger Natriumchloridlösung und trocknet sie über Natriumsulfat. Man filtriert und engt im Vakuum ein. Säulenchromatographie an Kieselgel mit einem Gemisch aus Hexan/Ethylacetat ergibt 248 mg der Titelverbindung als farblosen Schaum.
$^1$H-NMR ($CDCl_3$): δ= 7,18 d (J=9 Hz, 2H, aryl); 7,02 d (J=9 Hz, 2H, aryl); 5,79 s (1H, H-4); 4,45 dbr (J=6 Hz, 1H, H-11); 2,29 s (3H, acetyl); 0,61 s (3H, H-18).

**Beispiel 10**

17β-Hydroxy-11β-[4-(hydroxymethyl)phenyl]-17α-(1,1,2,2,2-pentafluorethyl)estra-4,9-dien-3-on

10a) 3,3-[2,2-Dimethyl-1,3-propandiylbis(oxy)]-5-hydroxy-11β-[4-[(methoxymethoxy)methyl]phenyl]-5α-estr-9-en-17-on

Aus 6,0 g 3,3-[2,2-Dimethyl-1,3-propandiylbis(oxy)]-5,10-epoxy-5a,10a-estr-9(11)-en-17-on in 160 ml absolutem Tetrahydrofuran, 22,32 g 1-Brom-4-[(methoxymethoxy)methyl]benzol (Herstellung siehe *Synth. Commun.* **20**, 1469-1472 (1990)) in 160 ml absolutem Tetrahydrofuran, 2,35 g Magnesiumspäne in 10 ml absolutem Tetrahydrofuran und 4,78 g Kupfer(I)chlorid erhält man analog zu dem unter 8a) beschriebenen Verfahren nach Säulenchromatographie an Kieselgel mit einem Gemisch aus Hexan/Ethylacetat 7,14 g der Titelverbindung als farblosen Schaum.
$^1$H-NMR ($CDCl_3$): δ=7,27 d (J=9 Hz, 2H, aryl); 7,24 d (J=9 Hz, 2H, aryl); 4,72 s (2H, acetal); 4,56 s (2H, benzyl); 4,48 s (1H, 5-OH); 4,33 dbr (J=6,5 Hz, 1H, H-11); 3,42 s (3H, methoxy); 1,07 s (3H, 3-ketal); 0,87 s (3H, 3-ketal); 0,49 s (3H, H-18).

10b) 3,3-[2,2-Dimethyl-1,3-propandiylbis(oxy)]-17α-(1,1,2,2,2-pentafluorethyl)-11β-[4-[(methoxymethoxy)methyl]phenyl]-5α-estr-9-en-5,17β-diol

Analog zu Beispiel 1c) werden 4,85 g der unter 10a) beschriebenen Verbindung in 200 ml absolutem Toluol mit dem aus 18,2 g 1-Iod-1,1,2,2,2-pentafluorethan und 43,3 ml einer 1,5 molaren Lösung von Methyllithium-Lithiumbromid-Komplex in Diethylether gebildeten Reagenz umgesetzt. Man erhält nach Säulenchromatographie an Kieselgel mit einem Gemisch aus Hexan/Ethylacetat 4,13 g der Titelverbindung als farblosen Schaum.
$^1$H-NMR ($CDCl_3$): δ= 7,25 d (J=9 Hz, 2H, aryl); 7,20 d (J=9 Hz, 2H, aryl); 4,71 s (2H, acetal); 4,54 s (2H, benzyl); 4,46 s (1H, 5-OH); 4,32 dbr (J=6 Hz, 1H, H-11); 3,41 s (3H, methoxy); 1,06 s (3H, 3-ketal); 0,86 s (3H, 3-ketal); 0,52 s (3H, H-18).

10c) 17β-Hydroxy-11β-[4-(hydroxymethyl)phenyl]-17α-(1,1,2,2,2-pentafluorethyl)estra-4,9-dien-3-on

4,13 g der unter 10b) beschriebenen Verbindung werden analog zu dem in 3b) beschriebenen Verfahren in 65 ml Methanol mit 2,84 ml wäßriger halbkonzentrierter Schwefelsäure zu 2,26 g der Titelverbindung als farblosem Schaum umgesetzt.
$^1$H-NMR ($CDCl_3$): δ= 7,27 d (J=9 Hz, 2H, aryl); 7,17 d (J=9 Hz, 2H, aryl); 5,78 s (1H, H-4); 4,64 s (2H, benzyl); 4,45 dbr (J=6,5 Hz, 1H, H-11); 0,59 s (3H, H-18).

**Beispiel 11**

4-[17β-Hydroxy-3-oxo-17α-(1,1,2,2,2-pentafluorethyl)estra-4,9-dien-11β-yl]benzaldehyd

497 mg der unter 10c) hergestellten Verbindung werden mit 431 mg Pyridiniumchlorochromat in 10 ml Dichlormethan zwei Stunden bei Raumtemperatur gerührt, dann über Kieselgel filtriert. Der Rückstand wird gründlich mit Ethylacetat nachgewaschen. Das Filtrat wird im Vakuum eingeengt. Nach Säulenchromatographie an Kieselgel mit einem Gemisch aus Hexan/Ethylacetat erhält man 415 mg der Titelverbindung als farblosen Schaum.
$^1$H-NMR ($CDCl_3$): δ=9,97 s (1H, formyl); 7,81 d (J=9 Hz, 2H, aryl); 7,39 d (J=9 Hz, 2H, aryl); 5,81 s (1H, H-4); 4,52 dbr (J=7 Hz, 1H, H-11); 0,58 s (3H, H-18).

**Beispiel 12**

4-[17β-Hydroxy-3-oxo-17α-(1,1,2,2,2-pentafluorethyl)estra-4,9-dien-11β-yl]-benzoesäuremethylester

Eine Lösung von 125 mg der unter 11 hergestellten Verbindung in 2,5 ml Methanol wird zu 81,4 mg Kaliumcyanid in 1,25 ml Methanol gegeben. 390 mg Mangan(IV)oxid und 22 ml Eisessig werden dem Reaktionsgemisch hinzugefügt, das sodann eine Stunde bei Raumtemperatur gerührt wird. Man filtriert über Celite® , nimmt das Filtrat in Ethylacetat/Wasser auf und extrahiert die wäßrige Phase mit Ethylacetat. Die vereinigten organischen Phasen werden mit Wasser und gesättigter wäßriger Natriumchloridlösung gewaschen, über Natriumsulfat getrocknet, filtriert und im Vakuum eingeengt. Man erhält nach Säulenchromatographie an Kieselgel mit einem Gemisch aus Hexan/Ethylacetat 120 mg der Titelverbindung als farblosen Schaum.
$^1$H-NMR ($CDCl_3$): δ= 7,94 d (J=9 Hz, 2H, aryl); 7,27 d (J=9 Hz, 2H, aryl); 5,79 s (1H, H-4); 4,49 dbr (J=6 Hz, 1H, H-11); 3,89 s (3H, methoxy); 0,57 s (3H, H-18).

**Beispiel 13**

17β-Hydroxy-11β-[4-(1-hydroxyethyl)phenyl]-17α-(1,1,2,2,2-pentafluorethyl)estra-4,9-dien-3-on

13a) 3,3-[2,2-Dimethyl-1,3-propandiylbis(oxy)]-5-hydroxy-11β-[4-[1-[(tetrahydro-2*H*-pyran-2-yl)oxy]ethyl]phenyl]-5α-estr-9-en-17-on

Aus 1,6 g 3,3-[2,2-Dimethyl-1,3-propandiylbis(oxy)]-5,10-epoxy-5α,10α-estr-9(11)-en-17-on in 40 ml absolutem Tetrahydrofuran, 7,4 g 2-[1-(4-Bromphenyl)ethoxy]tetrahydro-2*H*-pyran (Herstellung siehe *Arzneim. Forsch.* **25,** 1495-1501 (1975)) in 40 ml absolutem Tetrahydrofuran, 1,3 g Magnesiumspäne in 2 ml absolutem Tetrahydrofuran und 1,3 g Kupfer(I)chlorid erhält man analog zu dem unter 8a) beschriebenen Verfahren nach Säulenchromatographie an Kieselgel mit einem Gemisch aus Hexan/Ethylacetat 2,06 g der Titelverbindung als Diastereomerengemisch an der Acetal- und der Benzylposition.
$^1$H-NMR ($CDCl_3$): δ=7,28 d (J=9 Hz, 2H, aryl); 7,18 d (J=9 Hz, 2H, aryl); 4,90-4,72 m (2H, acetal und benzylether); 4,44 s (1H, 5-OH); 4,30 dbr (J=6,5 Hz, 1H, H-11); 1,45/1,42 d (J=6 Hz, 3H, methyl); 1,05 s (3H, 3-ketal); 0,87 s (3H, 3-ketal); 0,46 s (3H, H-18).

13b) 3,3-[2,2-Dimethyl-1,3-propandiylbis(oxy)]-17α-(1,1,2,2,2-pentafluorethyl)-11β-[4-[1-[(tetrahydro-2H-pyran-2-yl) oxy]ethyl]phenyl]-5α-estr-9-en-5,17β-diol

Analog zu Beispiel 1c) werden 1,45 g der unter 13a) beschriebenen Verbindung in 50 ml absolutem Toluol mit dem aus 4,9 g 1-Iod-1,1,2,2,2-pentafluorethan und 11,7 ml einer 1,5 molaren Lösung von Methyllithium-Lithiumbromid-Komplex in Diethylether gebildeten Reagenz umgesetzt. Man erhält nach Säulenchromatographie an Kieselgel mit einem Gemisch aus Hexan/Ethylacetat 1,22 g der Titelverbindung als Diastereomerengemisch an der Acetalund der Benzylposition.
$^1$H-NMR ($CDCl_3$): δ= 7,28 d (J=9 Hz, 2H, aryl); 7,18 d (J=9 Hz, 2H, aryl); 4,90-4,74 m (2H, acetal und benzylether); 4,42 s (1H, 5-OH); 4,31 dbr (J=6,5 Hz, 1H, H-11); 1,46/1,42 d (J=6 Hz, 3H, methyl); 1,05 s (3H, 3-ketal); 0,87 s (3H, 3-ketal); 0,51 s (3H, H-18).

13c) 17β-Hydroxy-11β-[4-(1-hydroxyethyl)phenyl]-17α-(1,1,2,2,2-pentafluorethyl)estra-4,9-dien-3-on

1,22 g der unter 13b) beschriebenen Verbindung werden analog zu dem in 3b) beschriebenen Verfahren in 18 ml Methanol mit 778 µl wäßriger halbkonzentrierter Schwefelsäure zu 693 mg der Titelverbindung als farblosem Schaum umgesetzt. Man erhält ein Epimerengemisch am Benzylcarbinol.
$^1$H-NMR ($CDCl_3$): δ=7,28 d (J=9 Hz, 2H, aryl); 7,15 d (J=9 Hz, 2H, aryl); 5,79 s (1H, H-4); 4,88 qbr (J=6 Hz, 1H, benzyl); 4,45 dbr (J=6 Hz, 1H, H-11); 1,49 d (J=6 Hz, 3H, methyl); 0,60 s (3H, H-18).

**Patentansprüche**

1. 17α-Fluoralkylsteroide der allgemeinen Formel I

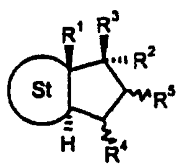

I

worin

$R^1$ für eine Methyl- oder Ethylgruppe,

$R^2$ für einen Rest der Formel $C_nF_mH_o$, wobei n 2, 3, 4, 5 oder 6, m>1 und m+o = 2n+1 ist,

$R^3$ für eine freie, veretherte oder veresterte Hydroxygruppe,

$R^4$ und $R^5$ je für ein Wasserstoffatom, gemeinsam für eine zusätzliche Bindung oder eine Methylengruppe,

St für ein steroidales ABC-Ringsystem der Teilformel A, B oder C

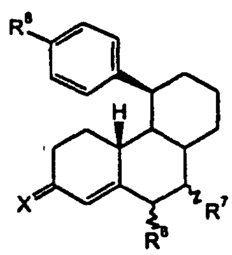

A

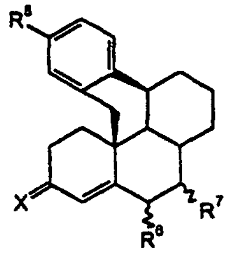

B

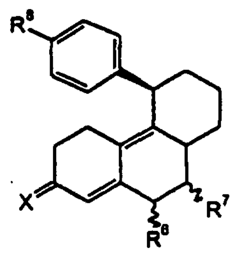

C

stehen,
worin

$R^6$ ein Wasserstoffatom, eine geradkettige $C_1$-$C_4$- oder verzweigte $C_3$-$C_4$-Alkylgruppe oder ein Halogenatom,

$R^7$ ein Wasserstoffatom, eine geradkettige $C_1$-$C_4$- oder eine verzweigte $C_3$-$C_4$-Alkylgruppe, oder, wenn St für ein steroidales ABC-Ringsystem A oder B steht, außerdem

$R^6$ und $R^7$ gemeinsam eine zusätzliche Bindung,

X ein Sauerstoffatom, eine Hydroxyiminogruppierung =N~OH oder zwei Wasserstoffatome,

$R^8$ einen Rest Y oder einen gegebenenfalls mehrfach mit einer Gruppe Y substituierten Arylrest, wobei Y ein Wasserstoffatom, ein Halogenatom, eine -OH, $-NO_2$, $-N_3$, -CN, $-NR^{9a}R^{9b}$, $-NHSO_2R^9$, $-CO_2R^9$, $C_1$-$C_{10}$-Alkyl-, $C_1$-$C_{10}$-Alkoxy-, $C_1$-$C_{10}$-Alkanoyloxy-, Benzoyloxy-, $C_1$-$C_{10}$-Alkanoyl-, $C_1$-$C_{10}$-Hydroxyalkyl- oder Benzoylgruppe ist und $R^{9a}$ und $R^{9b}$ gleich oder verschieden sind und wie $R^9$ ein Wasserstoffatom oder eine $C_1$-$C_{10}$-Alkylgruppe darstellen,

bedeuten,
sowie für die Reste-$NR^{9a}R^{9b}$ auch deren physiologisch verträgliche Salze mit Säuren und für die Reste $-CO_2R^9$ mit $R^9$ in der Bedeutung von Wasserstoff auch deren physiologisch verträgliche Salze mit Basen.

**2.** 17α-Fluoralkylsteroide nach Anspruch 1, worin St für ein steroidales Ringsystem der Teilformel A steht.

**3.** 17α-Fluoralkylsteroide nach Anspruch 1, worin St für ein steroidales Ringsystem der Teilformel B steht.

**4.** 17α-Fluoralkylsteroide nach Anspruch 1, worin St für ein steroidales Ringsystem der Teilformel C steht.

**5.** 17α-Fluoralkylsteroide nach Anspruch 1, worin n = 2, 3 oder 4 ist.

**6.** 17α-Fluoralkylsteroide nach Anspruch 5, worin o = 0 ist.

**7.** 17α-Fluoralkylsteroide nach Anspruch 6, worin n = 2 ist.

**8.** 17α-Fluoralkylsteroide nach Anspruch 1, worin $R^3$ eine freie Hydroxygruppe ist.

**9.** 17α-Fluoralkylsteroide nach Anspruch 1, worin $R^8$ für einen der Reste Y steht.

**10.** 17α-Fluoralkylsteroide nach Anspruch 9, worin Y für eine $C_1$-$C_{10}$-Acylgruppe steht.

**11.** 17α-Fluoralkylsteroide nach Anspruch 10, worin Y für eine Formyl-, Acetyl- oder Propionylgruppe steht.

**12.** 17α-Fluoralkylsteroide nach Anspruch 9, worin Y für eine $C_1$-$C_{10}$-Hydroxyalkylgruppe steht.

**13.** 17α-Fluoralkylsteroide nach Anspruch 12, worin Y für eine Hydroxymethyl- oder 1-Hydroxyethylgruppe steht.

**14.** 17α-Fluoralkylsteroide nach Anspruch 9, worin Y für eine Hydroxygruppe steht.

**15.** 17α-Fluoralkylsteroide nach Anspruch 9, worin Y für eine Acetyloxygruppe steht.

**16.** 17α-Fluoralkylsteroide nach Anspruch 9, worin Y für eine Methoxycarbonylgruppe steht.

**17.** 17α-Fluoralkylsteroide nach Anspruch 1, worin $R^8$ für einen mit einer Gruppe Y substituierten Arylrest steht.

**18.** 17α-Fluoralkylsteroide nach Anspruch 17, worin der Arylrest ein Phenyl-, Naphthalinyl-, Furanyl-, Benzofuranyl-, Thienyl- oder Pyridinylrest ist.

**19.** 17α-Fluoralkylsteroide nach Anspruch 18, worin $R^8$ ein 4-Cyanphenylrest ist.

**20.** 17α-Fluoralkylsteroide nach Anspruch 18, worin $R^8$ ein 4-Halogenphenylrest ist.

**21.** 17α-Fluoralkylsteroide nach Anspruch 20, worin $R^8$ ein 4-Fluorphenylrest ist.

**22.** 17α-Fluoralkylsteroide nach Anspruch 1, worin $R^4$ und $R^5$ je für ein Wasserstoffatom stehen.

**23.** 17α-Fluorakylsteroide nach Anspruch 1, worin $R^4$ und $R^5$ gemeinsam für eine zusätzliche Bindung stehen.

**24.** 17α-Fluoralkylsteroide nach Anspruch 1, worin $R^6$ und $R^7$ je ein Wasserstoffatom sind.

**25.** 17α-Fluoralkylsteroide nach Anspruch 1, nämlich

11β-(4-Acetylphenyl)-17β-hydroxy-17α-(1,1,2,2,2-pentafluorethyl)estr-4-en-3-on;

4'-[17β-Hydroxy-3-oxo-17α-(1,1,2,2,2-pentafluorethyl)estr-4-en-11β-yl][1,1'-biphenyl]-4-carbonitril;

11β-(4'-Fluor[1,1'-biphenyl]-4-yl)-17β-hydroxy-17α-(1,1,2,2,2-pentafluorethyl)estr-4-en-3-on;

17β-Hydroxy-17α-(1,1,2,2,2-pentafluorethyl)-11β-[4-(3-pyridinyl)phenyl]estr-4-en-3-on;

11β-(4-Acetylphenyl)-17β-hydroxy-17α-(1,1,2,2,2-pentafluorethyl)estra-4,15-dien-3-on;

4'-[17β-Hydroxy-3-oxo-17α-(1,1,2,2,2-pentafluorethyl)estra-4,15-dien-11β-yl][1,1'-biphenyl]-4-carbonitril;

11β-(4'-Fluor[1,1'-biphenyl]-4-yl)-17β-hydroxy-17α-(1,1,2,2,2-pentafluorethyl)estra-4,15-dien-3-on;

17β-Hydroxy-17α-(1,1,2,2,2-pentafluorethyl)-11β-[4-(3-pyridinyl)phenyl]estra-4,15-dien-3-on;

11β-(4-Acetylphenyl)-17β-hydroxy-17α-(1,1,2,2,2-pentafluorethyl)estra-4,9-dien-3-on;

4'-[17β-Hydroxy-3-oxo-17α-(1,1,2,2,2-pentafluorethyl)estra-4,9-dien-11β-yl][1,1'-biphenyl]-4-carbonitril;

11β-(4'-Fluor[1,1'-biphenyl]-4-yl)-17β-hydroxy-17α-(1,1,2,2,2-pentafluorethyl)estra-4,9-dien-3-on;

17β-Hydroxy-17α-(1,1,2,2,2-pentafluorethyl)-11β-[4-(3-pyridinyl)phenyl]estra-4,9-dien-3-on;

11β-(4-Acetylphenyl)-17β-hydroxy-17α-(1,1,2,2,2-pentafluorethyl)estra-4,9,15-trien-3-on;

4'-[17β-Hydroxy-3-oxo-17α-(1,1,2,2,2-pentafluorethyl)estra-4,9,15-trien-11β-yl][1,1'biphenyl]-4-carbonitril;

11β-(4'-Fluor[1,1'-biphenyl]-4-yl)-17β-hydroxy-17α-(1,1,2,2,2-pentafluorethyl))estra-4,9,15-trien-3-on;

17β-Hydroxy-17α-(1,1,2,2,2-pentafluorethyl)-11β-[4-(3-pyridinyl)phenyl]estra-4,9,15-trien-3-on;

6'-Acetyl-9,11α-dihydro-17β-hydroxy-17α-(1,1,2,2,2-pentafluorethyl)-4'*H*naphth[3',2',1':10,9,11]estr-4-en-3-on;

4-[9,11α-dihydro-17β-hydroxy-3-oxo-17α-(1,1,2,2,2-pentafluorethyl)-4'*H*naphth[3',2',1':10,9,11]estr-4-en-6'-yl]benzonitril;

9,11α-Dihydro-6'-(4-fluorphenyl)-17β-hydroxy-17α-(1,1,2,2,2-pentafluorethyl)-4'*H*naphth[3',2',1':10,9,11]estr-4-en-3-on;

9,11α-Dihydro-17β-hydroxy-17α-(1,1,2,2,2-pentafluorethyl)-6'-(3-pyridinyl)-4'*H*naphth[3',2',1':10,9,11]estr-4-en-3-on;

6'-Acetyl-9,11α-dihydro-17β-hydroxy-17α-(1,1,2,2,2-pentafluorethyl)-4'*H*naphth[3',2',1':10,9,11]estra-4,15-dien-3-on;

4-[9,11α-dihydro-17β-hydroxy-3-oxo-17α-(1,1,2,2,2-pentafluorethyl)-4'*H*naphth[3',2',1':10,9,11]estra-4,15-dien-6'-yl]benzonitril;

9,11α-Dihydro-6'-(4-fluorphenyl)-17β-hydroxy-17α-(1,1,2,2,2-pentafluorethyl)-4'*H*naphth[3',2',1':10,9,11] estra-4,15-dien-3-on;

9,11α-Dihydro-17β-hydroxy-17α-(1,1,2,2,2-pentafluorethyl)-6'-(3-pyridinyl)-4'*H*naphth[3',2',1':10,9,11]estra-4,15-dien-3-on;

17β-Hydroxy-11β-(4-hydroxyphenyl)-17α-(1,1,2,2,2-pentafluorethyl)estra-4,9-dien-3-on;

17β-Hydroxy-11β-(4-hydroxyphenyl)-17α-(1,1,2,2,2-pentafluorethyl)estr-4-en-3-on;

9,11α-Dihydro-6',17β-dihydroxy-17α-(1,1,2,2,2-pentafluorethyl)-4'*H*naphth[3',2',1': 10,9,11]estr-4-en-3-on;

11 β-[4-(Acetyloxy)phenyl]-17β-hydroxy-17α-(1,1,2,2,2-pentafluorethyl)estra-4,9-dien-3-on;

11β-[4-(Acetyloxy)phenyl]-17β-hydroxy-17α-(1,1,2,2,2-pentafluorethyl)estr-4-en-3-on

6'-(Acetyloxy)-9,11α-dihydro-17β-hydroxy-17α-(1,1,2,2,2-pentafluorethyl)-4'*H*-naphth[3',2',1':10,9,11]estr-4-en-3-on;

17β-Hydroxy-11β-[4-(hydroxymethyl)phenyl]-17α-(1,1,2,2,2-pentafluorethyl)estra-4,9-dien-3-on;

17β-Hydroxy-11β-[4-(hydroxymethyl)phenyl]-17α-(1,1,2,2,2-pentafluorethyl)estr-4-en-3-on;

9,11α-Dihydro-17β-hydroxy-6'-(hydroxymethyl)-17α-(1,1,2,2,2-pentafluorethyl)-4'*H*naphth[3',2',1':10,9,11] estr-4-en-3-on;

4-[17β-Hydroxy-3-oxo-17α-(1,1,2,2,2-pentafluorethyl)estra-4,9-dien-11β-yl]benzaldehyd;

4-[17β-Hydroxy-3-oxo-17α-(1,1,2,2,2-pentafluorethyl)estr-4-en-11β-yl]benzaldehyd;

9,11α-Dihydro-17β-hydroxy-3-oxo-17α-(1,1,2,2,2-pentafluorethyl)-4'*H*naphth[3',2',1':10,9,11]estr-4-en-6'-al;

4-[17β-Hydroxy-3-oxo-17α-(1,1,2,2,2-pentafluorethyl)estra-4,9-dien-11β-yl]benzoesäuremethylester;

4-[17β-Hydroxy-3-oxo-17α-(1,1,2,2,2-pentafluorethyl)estra-4-en-11β-yl]benzoesäuremethylester;

9,11α-Dihydro-17β-hydroxy-3-oxo-17α-(1,1,2,2,2-pentafluorethyl)-4'*H*naphth[3',2',1':10,9,11]estr-4-en-6'-carbonsäuremethylester;

17β-Hydroxy-11β-[4-(1-hydroxyethyl)phenyl]-17α-(1,1,2,2,2-pentafluorethyl)estra-4,9-dien-3-on;

17β-Hydroxy-11β-[4-(1-hydroxyethyl)phenyl]-17α-(1,1,2,2,2-pentafluorethyl)estr-4-en-3-on;

9,11α-Dihydro-17β-hydroxy-6'-(1-hydroxyethyl)-17α-(1,1,2,2,2-pentafluorethyl)-4'*H*naphth[3',2',1':10,9,11] estr-4-en-3-on.

**26.** Pharmazeutische Präparate, enthaltend mindestens ein 17α-Fluoralkylsteroid der allgemeinen Formel I gemäß Anspruch 1 sowie einen pharmazeutisch verträglichen Träger.

**27.** Pharmazeutische Präparate nach Anspruch 26, enthaltend zusätzlich mindestens eine Verbindung mit antiestrogener Wirkung.

**28.** Verwendung der 17α-Fluoralkylsteroide der allgemeinen Formel I gemäß Anspruch 1 zur Herstellung von Arzneimitteln.

**29.** Verwendung nach Anspruch 28 zusätzlich mit einer Verbindung mit antiestrogener Wirkung.

## Claims

**1.** 17α-Fluoroalkyl steroids of general formula I

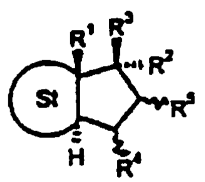

I

in which

$R^1$ stands for a methyl or ethyl group,
$R^2$ stands for a radical of formula $C_nF_mH_o$, where n is 2, 3, 4, 5 or 6, m > 1 and m+o = 2n+1,
$R^3$ stands for a free etherified or esterified hydroxyl group,
$R^4$ and $R^5$ each stand for a hydrogen atom, together for an additional bond or a methylene group,
St stands for a steroidal ABC ring system of partial formula A, B or C

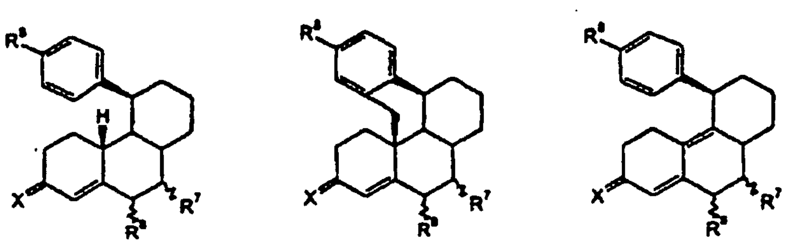

A B C

in which
$R^6$ means a hydrogen atom, a straight-chain $C_1$-$C_4$ alkyl group or branched $C_3$-$C_4$ alkyl group or a halogen atom,

$R^7$ means a hydrogen atom, a straight-chain $C_1$-$C_4$ alkyl group or a branched $C_3$-$C_4$ alkyl group, or

if St stands for a steroidal ABC ring system A or B, in addition

$R^6$ and $R^7$ together mean an additional bond,
X means an oxygen atom, a hydroxyimino grouping =N-OH or two hydrogen atoms,
$R^8$ means a radical Y or an aryl radical that is optionally substituted several times with a group Y, where Y is a hydrogen atom, a halogen atom, an -OH, $-NO_2$, $-N_3$, -CN, $-NR^{9a}R^{9b}$, $-NHSO_2R^9$, $-CO_2R^9$, $C_1$-$C_{10}$ alkyl, $C_1$-$C_{10}$ alkoxy, $C_1$-$C_{10}$ alkanoyloxy, benzoyloxy, $C_1$-$C_{10}$ alkanoyl, $C_1$-$C_{10}$ hydroxyalkyl or benzoyl group, and
$R^{9a}$ and $R^{9b}$ are the same or different and in the same way as $R^9$ represent a hydrogen atom or a $C_1$-$C_{10}$ alkyl group,

and, for $-NR^{9a}R^{9b}$ radicals, also their physiologically compatible salts with acids and, for $-CO_2R^9$ radicals with $R^9$ meaning hydrogen, also their physiologically compatible salts with bases.

**2.** 17α-Fluoroalkyl steroids according to claim 1, in which St stands for a steroidal ring system of partial formula A.

**3.** 17α-Fluoroalkyl steroids according to claim 1, in which St stands for a steroidal ring system of partial formula B.

**4.** 17α-Fluoroalkyl steroids according to claim 1, in which St stands for a steroidal ring system of partial formula C.

**5.** 17α-Fluoroalkyl steroids according to claim 1, in which n = 2, 3 or 4.

**6.** 17α-Fluoroalkyl steroids according to claim 5, in which o = 0.

**7.** 17α-Fluoroalkyl steroids according to claim 6, in which n = 2.

**8.** 17α-Fluoroalkyl steroids according to claim 1, in which $R^3$ is a free hydroxyl group.

**9.** 17α-Fluoroalkyl steroids according to claim 1, in which $R^8$ stands for one of the radicals Y.

**10.** 17α-Fluoroalkyl steroids according to claim 9, in which Y stands for a $C_1$-$C_{10}$ acyl group.

**11.** 17α-Fluoroalkyl steroids according to claim 10, in which Y stands for a formyl, acetyl or propionyl group.

**12.** 17α-Fluoroalkyl steroids according to claim 9, in which Y stands for a $C_1$-$C_{10}$ hydroxyalkyl group.

**13.** 17α-Fluoroalkyl steroids according to claim 12, in which Y stands for a hydroxymethyl or 1-hydroxyethyl group.

**14.** 17α-Fluoroalkyl steroids according to claim 9, in which Y stands for a hydroxyl group.

**15.** 17α-Fluoroalkyl steroids according to claim 9, in which Y stands for an acetyloxy group.

**16.** 17α-Fluoroalkyl steroids according to claim 9, in which Y stands for a methoxycarbonyl group.

**17.** 17α-Fluoroalkyl steroids according to claim 1, in which $R^8$ stands for an aryl radical that is substituted with a group Y.

**18.** 17α-Fluoroalkyl steroids according to claim 17, in which the aryl radical is a phenyl, naphthalenyl, furanyl, benzofuranyl, thienyl or pyridinyl radical.

**19.** 17α-Fluoroalkyl steroids according to claim 18, in which $R^8$ is a 4-cyanophenyl radical.

**20.** 17α-Fluoroalkyl steroids according to claim 18, in which $R^8$ is a 4-halophenyl radical.

**21.** 17α-Fluoroalkyl steroids according to claim 20, in which $R^8$ is a 4-fluorophenyl radical.

**22.** 17α-Fluoroalkyl steroids according to claim 1, in which $R^4$ and $R^5$ each stand for a hydrogen atom.

**23.** 17α-Fluoroalkyl steroids according to claim 1, in which $R^4$ and $R^5$ together stand for an additional bond.

**24.** 17α-Fluoroalkyl steroids according to claim 1, in which $R^6$ and $R^7$ are each a hydrogen atom.

**25.** 17α-Fluoroalkyl steroids according to claim 1, namely 11β-(4-acetylphenyl)-17β-hydroxy-17α-(1,1,2,2,2-pentafluoroethyl)estr-4-en-3-one;

4'-[17β-hydroxy-3-oxo-17α-(1,1,2,2,2-pentafluoroethyl)estr-4-en-11β-yl][1,1'-biphenyl]-4-carbonitrile;
11β-(4'-fluoro[1,1'-biphenyl]-4-yl)-17β-hydroxy-17α-(1,1,2,2,2-pentafluoroethyl)estr-4-en-3-one;
17β-hydroxy-17α-(1,1,2,2,2-pentafluoroethyl)-11β-[4-(3-pyridinyl)phenyl]estr-4-en-3-one;
11β-(4-acetylphenyl)-17β-hydroxy-17α-(1,1,2,2,2-pentafluoroethyl)estra-4,15-dien-3-one;
4'-[17β-hydroxy-3-oxo-17α-(1,1,2,2,2-pentafluoroethyl)estra-4,15-dien-11β-yl][1,1'-biphenyl]-4-carbonitrile;
11β-(4'-fluoro[1,1'-biphenyl]-4-yl)-17β-hydroxy-17α-(1,1,2,2,2-pentafluoroethyl)estra-4,15-dien-3-one;
17β-hydroxy-17α-(1,1,2,2,2-pentafluoroethyl)-11β-[4-(3-pyridinyl)phenyl]estra-4,15-dien-3-one;
11β-(4-acetylphenyl)-17β-hydroxy-17α-(1,1,2,2,2-pentafluoroethyl)estra-4,9-dien-3-one;
4'-[17β-hydroxy-3-oxo-17α-(1,1,2,2,2-pentafluoroethyl)estra-4,9-dien-11β-yl][1,1'-biphenyl]-4-carbonitrile;
11β-(4'-fluoro[1,1'-biphenyl]-4-yl)-17β-hydroxy-17α-(1,1,2,2,2-pentafluoroethyl)estra-4,9-dien-3-one;
17β-hydroxy-17α-(1,1,2,2,2-pentafluoroethyl)-11β-[4- (3-pyridinyl)phenyl]estra-4,9-dien-3-one;
11β-(4-acetylphenyl)-17β-hydroxy-17α-(1,1,2,2,2-pentafluoroethyl)estra-4,9,15-trien-3-one;
4'-[17β-hydroxy-3-oxo-17α-(1,1,2,2,2-pentafluoroethyl)estra-4,9,15-trien-11β-yl][1,1'-biphenyl]-4-carbonitrile;
11β-(4'-fluoro(1,1'-biphenyl]-4-yl)-17β-hydroxy-17α-(1,1,2,2,2-pentafluoroethyl))estra-4,9,15-trien-3-one;
17β-hydroxy-17α-(1,1,2,2,2-pentafluoroethyl)-11β-[4-(3-pyridinyl)phenyl]estra-4,9,15-trien-3-one;
6'-acetyl-9,11α-dihydro-17β-hydroxy-17α-(1,1,2,2,2-pentafluoroethyl)-4'H-naphth-[3',2',1':10,9,11]estr-4-en-3-one;
4-[9,11α-dihydro-17β-hydroxy-3-oxo-17α-(1,1,2,2,2-pentafluoroethyl)-4'H-naphth[3',2',1':10,9,11]estr-4-en-6'-yl]benzonitrile;
9,11α-dihydro-6'-(4-fluorophenyl)-17β-hydroxy-17α-(1,1,2,2,2-pentafluoroethyl)-4'H-naphth-[3',2',1':10,9,11] estr-4-en-3-one;
9,11α-dihydro-17β-hydroxy-17α-(1,1,2,2,2-pentafluoroethyl)-6'-(3-pyridinyl)-4'H-naphth[3',2',1':10,9,11]estr-4-en-3-one;
6'-acetyl-9,11α-dihydro-17β-hydroxy-17α-(1,1,2,2,2-pentafluoroethyl)-4'H-naphth-[3',2',1':10,9,11]estra-4,15-dien-3-one;
4-[9,11α-dihydro-17β-hydroxy-3-oxo-17α-(1,1,2,2,2-pentafluoroethyl)-4'H-naphth[3',2',1':10,9,11]estra-4,15-dien-6'-yl]benzonitrile;
9,11α-dihydro-6'-(4-fluorophenyl)-17β-hydroxy-17α-(1,1,2,2,2-pentafluoroethyl)-4'H-naphth-[3',2',1':10,9,11] estra-4,15-dien-3-one;
9,11α-dihydro-17β-hydroxy-17α-(1,1,2,2,2-pentafluoroethyl)-6'-(3-pyridinyl)-4'H-naphth[3',2',1':10,9,11]estra-4,15-dien-3-one;
17β-hydroxy-11β-(4-hydroxyphenyl)-17α-(1,1,2,2,2-pentafluoroethyl)estra-4,9-dien-3-one;
17β-hydroxy-11β-(4-hydroxyphenyl)-17α-(1,1,2,2,2-pentafluoroethyl)estr-4-en-3-one;
9,11α-dihydro-6',17β-dihydroxy-17α-(1,1,2,2,2-pentafluoroethyl)-4'H-naphth[3',2',1':10,9,11]estr-4-en-3-one;
11β-[4-(acetyloxy)phenyl]-17β-hydroxy-17α-(1,1,2,2,2-pentafluoroethyl)estra-4,9-dien-3-one;
11β-[4-(acetyloxy)phenyl]-17β-hydroxy-17α-(1,1,2,2,2-pentafluoroethyl)estr-4-en-3-one
6'-acetyloxy-9,11α-dihydro-17β-hydroxy-17α-(1,1,2,2,2-pentafluoroethyl)-4'H-naphth-[3',2',1':10,9,11]estr-4-en-3-one;
17β-hydroxy-11β-[4-(hydroxymethyl)phenyl]-17α-(1,1,2,2,2-pentafluoroethyl)estra-4,9-dien-3-one;
17β-hydroxy-11β-[4-(hydroxymethyl)phenyl]-17α-(1,1,2,2,2-pentafluoroethyl)estr-4-en-3-one;
9,11α-dihydro-17β-hydroxy-6'-hydroxymethyl-17α-(1,1,2,2,2-pentafluoroethyl)-4'H-naphth[3',2',1':10,9,11] estr-4-en-3-one;
4-[17β-hydroxy-3-oxo-17α-(1,1,2,2,2-pentafluoroethyl)estra-4,9-dien-11β-yl]benzaldehyde;
4-[17β-hydroxy-3-oxo-17α-(1,1,2,2,2-pentafluoroethyl)estr-4-en-11β-yl]benzaldehyde;
9,11α-dihydro-17β-hydroxy-3-oxo-17α-(1,1,2,2,2-pentafluoroethyl)-4'H-naphth[3',2',1':10,9,11]estr-4-en-6'-al;
methyl 4-[17β-hydroxy-3-oxo-17α-(1,1,2,2,2-pentafluoroethyl)estra-4,9-dien-11β-yl]benzoate;
methyl 4-[17β-hydroxy-3-oxo-17α-(1,1,2,2,2-pentafluoroethyl)estr-4-en-11β-yl]benzoate;
methyl 9,11α-dihydro-17β-hydroxy-3-oxo-17α-(1,1,2,2,2-pentafluoroethyl)-4'H-naphth-[3',2',1':10,9,11]estr-4-en-6'-carboxylate;
17β-hydroxy-11β-[4-(1-hydroxyethyl)phenyl]-17α-(1,1,2,2,2-pentafluoroethyl)estra-4,9-dien-3-one;

17β-hydroxy-11β-[4-(1-hydroxyethyl)phenyl]-17α-(1,1,2,2,2-pentafluoroethyl)estr-4-en-3-one;
9,11α-dihydro-17β-hydroxy-6'-(1-hydroxyethyl)-17α-(1,1,2,2,2-pentafluoroethyl)-4'H-naphth-[3',2',1':10,9,11] estr-4-en-3-one.

**26.** Pharmaceutical preparations that contain at least one 17α-fluoroalkyl steroid of general formula I according to claim 1 as well as a pharmaceutically compatible vehicle.

**27.** Pharmaceutical preparations according to claim 26 that additionally contain at least one compound with antiestrogenic action.

**28.** Use of the 17α-fluoroalkyl steroids of general formula I according to claim 1 for the preparation of pharmaceutical agents.

**29.** Use according to claim 28 additionally with a compound with antiestrogenic action.

**Revendications**

**1.** 17α-fluoroalkylstéroïdes de formule générale I

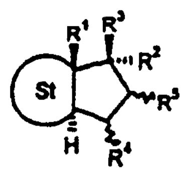

I

dans laquelle

R1 représente le groupe méthyle ou éthyle,
R2 représente un radical de formule CnFmHo, n étant 2, 3, 4, 5 ou 6, m étant >1 et m+o = 2n+1,
R3 représente un groupe hydroxy éthérifié ou estérifié,
R4 et R5 représentent chacun un atome d'hydrogène, ou forment ensemble une liaison supplémentaire ou un groupe méthylène,
St représente un système cyclique stéroïdien ABC de formule partielle A, B ou C

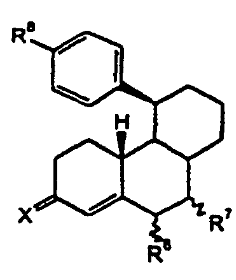

A

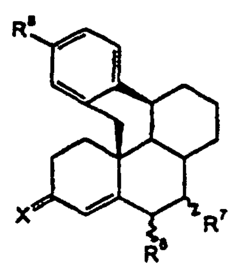

B

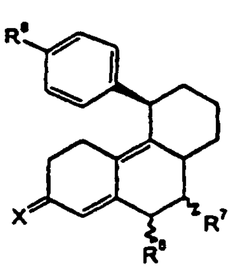

C

formules dans lesquelles

R6 représente un atome d'hydrogène, un groupe alkyle en C1-C4 à chaîne droite ou en C3-C4 ramifié ou un atome d'halogène,
R7 représente un atome d'hydrogène, un groupe alkyle en C1-C4 à chaîne droite ou en C3-C4 ramifié,

ou
lorsque St représente un système cyclique stéroïdien ABC A ou B, en outre,

R6 et R7 forment ensemble une liaison supplémentaire,
X représente un atome d'oxygène, un groupement hydroxyimino =N~OH ou deux atomes d'hydrogène,
R8 représente un radical Y ou un radical aryle éventuellement plusieurs fois substitué par un groupe Y, Y représentant un atome d'hydrogène, un atome d'halogène, un groupe -OH, -NO2, -N3, -CN, -NR9aR9b, $-NHSO_2R9$, -CO2R9, alkyle en C1-C10, alcoxy en C1-C10, alcanoyloxy en C1-C10, benzyloxy, alcanoyle en C1-C10, hydroxyalkyle en C1-C10 ou benzoyle, et R9a et R9b étant identiques ou différents et, comme R9, représentant un atome d'hydrogène ou un groupe alkyle en C1-C10,

ainsi que, pour les radicaux -NR9aR9b, également leurs sels physiologiquement acceptables avec des acides, et, pour les radicaux -CO2R9 où R9 représente un atome d'hydrogène, également leurs sels physiologiquement acceptables avec des bases.

**2.** 17α-fluoroalkylstéroïdes selon la revendication 1, dans lesquels St représente un système cyclique stéroïdien de formule partielle A.

**3.** 17α-fluoroalkylstéroïdes selon la revendication 1, dans lesquels St représente un système cyclique stéroïdien de formule partielle B.

**4.** 17α-fluoroalkylstéroïdes selon la revendication 1, dans lesquels St représente un système cyclique stéroïdien de formule partielle C.

**5.** 17α-fluoroalkylstéroïdes selon la revendication 1, dans lesquels n = 2, 3 ou 4.

**6.** 17α-fluoroalkylstéroïdes selon la revendication 5, dans lesquels o = 0.

**7.** 17α-fluoroalkylstéroïdes selon la revendication 6, dans lesquels n = 2.

**8.** 17α-fluoroalkylstéroïdes selon la revendication 1, dans lesquels R3 est le groupe hydroxy.

**9.** 17α-fluoroalkylstéroïdes selon la revendication 1, dans lesquels R8 représente l'un des radicaux Y.

**10.** 17α-fluoroalkylstéroïdes selon la revendication 9, dans lesquels Y représente un groupe acyle en C1-C10.

**11.** 17α-fluoroalkylstéroïdes selon la revendication 10, dans lesquels Y représente le groupe formyle, acétyle ou propionyle.

**12.** 17α-fluoroalkylstéroïdes selon la revendication 9, dans lesquels Y représente un groupe hydroxyalkyle en C1-C10.

**13.** 17α-fluoroalkylstéroïdes selon la revendication 12, dans lesquels Y représente le groupe hydroxyméthyle ou 1-hydroxyéthyle.

**14.** 17α-fluoroalkylstéroïdes selon la revendication 9, dans lesquels Y représente le groupe hydroxy.

**15.** 17α-fluoroalkylstéroïdes selon la revendication 9, dans lesquels Y représente le groupe acétyloxy.

**16.** 17α-fluoroalkylstéroïdes selon la revendication 9, dans lesquels Y représente le groupe méthoxycarbonyle.

**17.** 17α-fluoroalkylstéroïdes selon la revendication 1, dans lesquels R8 représente un radical aryle substitué par un groupe Y.

**18.** 17α-fluoroalkylstéroïdes selon la revendication 17, dans lesquels le radical aryle est le groupe phényle, naphtalinyle, furannyle, benzofurannyle, thiényle ou pyridinyle.

**19.** 17α-fluoroalkylstéroïdes selon la revendication 18, dans lesquels R8 est le groupe 4-cyanophényle.

**20.** 17α-fluoroalkylstéroïdes selon la revendication 18, dans lesquels R8 est un groupe 4-halogénophényle.

**21.** 17α-fluoroalkylstéroïdes selon la revendication 20, dans lesquels R8 est le groupe 4-fluorophényle.

**22.** 17α-fluoroalkylstéroïdes selon la revendication 1, dans lesquels R4 et R5 représentent chacun un atome d'hydrogène.

**23.** 17α-fluoroalkylstéroïdes selon la revendication 1, dans lesquels R4 et R5 forment ensemble une liaison supplémentaire.

**24.** 17α-fluoroalkylstéroïdes selon la revendication 1, dans lesquels R6 et R7 représentent chacun un atome d'hydrogène.

**25.** 17α-fluoroalkylstéroïdes selon la revendication 1, à savoir

11β-(4-acétylphényl)-17β-hydroxy-17α-(1,1,2,2,2-pentafluoroéthyl)estr-4-én-3-one,
4'-[17β-hydroxy-3-oxo-17α-(1,1,2,2,2-pentafluoroéthyl)estr-4-én-11β-yl] [1,1'-biphényl]-4-carbonitrile,
11β-(4'-fluoro[1,1'-biphényl]-4-yl)-17β-hydroxy-17α-(1,1,2,2,2-pentafluoroéthyl)estr-4-én-3-one,
17β-hydroxy-17α-(1,1,2,2,2-pentafluoroéthyl)-11β-[4-(3-pyridinyl)phényl)estr-4-én-3-one,
11β-(4-acétylphényl)-17β-hydroxy-17α-(1,1,2,2,2-pentafluoroéthyl)estra-4,15-dién-3-one,
4'-[17β-hydroxy-3-oxo-17α-(1,1,2,2,2-pentafluoroéthyl)estra-4,15-dién-11β-yl]-[1,1'-biphényl]-4-carbonitrile,
11β-(4'-fluoro[1,1'-biphényl]-4-yl)-17β-hydroxy-17α-(1,1,2,2,2-pentafluoroéthyl)estra-4,15-dién-3-one,
17β-hydroxy-17α-(1,1,2,2,2-pentafluoroéthyl)-11β-[4-(3-pyridinyl)phényl]estra-4,15-dién-3-one,
11β-(4-acétylphényl)-17β-hydroxy-17α-(1,1,2,2,2-pentafluoroéthyl)estra-4,9-dién-3-one,
4'-[17β-hydroxy-3-oxo-17α-(1,1,2,2,2-pentafluoroéthyl)estra-4,9-dién-11β-yl][1,1'-biphényl]-4-carbonitrile,
11β-(4'-fluoro[1,1'-biphényl]-4-yl)-17β-hydroxy-17α-(1,1,2,2,2-pentafluoroéthyl)estra-4,9-dién-3-one,
17β-hydroxy-17α-(1,1,2,2,2-pentafluoroéthyl)-11β-[4-(3-pyridinyl)phényl]estra-4,9-dién-3-one,
11β-(4-acétylphényl)-17β-hydroxy-17α-(1,1,2,2,2-pentafluoroéthyl)estra-4,9,15-trién-3-one,
4'-[17β-hydroxy-3-oxo-17α-(1,1,2,2,2-pentafluoroéthyl)estra-4,9,15-trién-11β-yl][1,1'-biphényl]-4-carbonitrile,
11β-(4'-fluoro[1,1'-biphényl]-4-yl)-17β-hydroxy-17α-(1,1,2,2,2-pentafluoroéthyl)estra-4,9,15-trién-3-one,
17β-hydroxy-17α-(1,1,2,2,2-pentafluoroéthyl)-11β-[4- (3-pyridinyl)phényl]estra-4,9,15-trién-3-one,
6'-acétyl-9,11α-hydro-17β-hydroxy-17α-(1,1,2,2,2-pentafluoroéthyl)-4'H-napht-[3',2',1':10,9,11]estr-4-én-3-one,
4-[9,11α-dihydro-17β-hydroxy-3-oxo-17α-(1,1,2,2,2-pentafluoroéthyl)-4'H-napht-[3',2',1':10,9,11]estr-4-én-6'-yl]benzonitrile,
9,11α-dihydro-6'-(4-fluorophényl)-17β-hydroxy-17α-(1,1,2,2,2-pentafluoroéthyl)-4'H-napht-[3',2',1':10,9,11] estr-4-én-3-one,
9,11α-dihydro-17β-hydroxy-17α-(1,1,2,2,2-pentafluoroéthyl)-6'-(3-pyridinyl)-4'H-napht-[3',2',1':10,9,11]estr-4-én-3-one,
6'-acétyl-9,11α-dihydro-17β-hydroxy-17α-(1,1,2,2,2-pentafluoroéthyl)-4'H-napht-[3',2',1':10,9,11]estra-4,15-dién-3-one,
4-[9,11α-dihydro-17β-hydroxy-3-oxo-17α-(1,1,2,2,2-pentafluoroéthyl)-4'H-napht-[3',2',1':10,9,11]estra-4,15-dién-6'-yl]benzonitrile,
9,11α-dihydro-6'-(4-fluorophényl)-17β-hydroxy-17α-(1,1,2,2,2-pentafluoroéthyl)-4'H-napht-[3',2',1':10,9,11] estra-4,15-dién-3-one,
9,11α-dihydro-17β-hydroxy-17α-(1,1,2,2,2-pentafluoroéthyl)-6'-(3-pyridinyl)-4'H-napht-[3',2',1':10,9,11]estra-4,15-dién-3-one,
17β-hydroxy-11β-(4-hydroxyphényl)-17α-(1,1,2,2,2-pentafluoroéthyl)estra-4,9-dién-3-one,
17β-hydroxy-11β-(4-hydroxyphényl)-17α-(1,1,2,2,2-pentafluoroéthyl)estr-4-én-3-one,
9,11α-dihydro-6',17β-dihydroxy-17α-(1,1,2,2,2-pentafluoroéthyl)-4'H-napht-[3',2',1':10,9,11]estr-4-én-3-one,
11β-[4-(acétyloxy)phényl]-17β-hydroxy-17α-(1,1,2,2,2-pentafluoroéthyl)estra-4,9-dién-3-one,
11β-[4-(acétyloxy)phényl]-17β-hydroxy-17α-(1,1,2,2,2-pentafluoroéthyl)estr-4-én-3-one,
6'-(acétyloxy)-9,11α-dihydro-17β-hydroxy-17α-(1,1,2,2,2-pentafluoroéthyl)-4'H-napht[3',2',1':10,9,11]estr-4-én-3-one,
17β-hydroxy-11β-[4-(hydroxyméthyl)phényl])-17α-(1,1,2,2,2-pentafluoroéthyl)estra-4,9-dién-3-one,
17β-hydroxy-11β-[4-(hydroxyméthyl)phényl]-17α-(1,1,2,2,2-pentafluoroéthyl)estr-4-én-3-one,
9,11α-dihydro-17β-hydroxy-6'-(hydroxyméthyl)-17α-(1,1,2,2,2-pentafluoroéthyl)-4'H-napht-[3',2',1':10,9,11] estr-4-én-3-one,

4-[17β-hydroxy-3-oxo-17α-(1,1,2,2,2-pentafluoroéthyl)estra-4,9-dién-11β-yl]-benzaldéhyde,
4-[17β-hydroxy-3-oxo-17α-(1,1,2,2,2-pentafluoroéthyl)estr-4-én-11β-yl]-benzaldéhyde,
9,11α-dihydro-17β-hydroxy-3-oxo-17α-(1,1,2,2,2-pentafluoroéthyl)-4'H-napht-[3',2',1':10,9,11]estr-4-én-6'-al,
4-[17β-hydroxy-3-oxo-17α-(1,1,2,2,2-pentafluoroéthyl)estra-4,9-dién-11β-yl]-benzoate de méthyle,
4-[17β-hydroxy-3-oxo-17α-(1,1,2,2,2-pentafluoroéthyl)estra-4-én-11β-yl]-benzoate de méthyle,
9,11α-dihydro-17β-hydroxy-3-oxo-17α-(1,1,2,2,2-pentafluoroéthyl)-4'H-napht-[3',2',1':10,9,11]estr-4-én-6'-carboxylate de méthyle,
17β-hydroxy-11β-[4-(1-hydroxyéthyl)phényl]-17α-(1,1,2,2,2-pentafluoroéthyl)estra-4,9-dién-3-one,
17β-hydroxy-11β-[4-(1-hydroxyéthyl)phényl]-17α-(1,1,2,2,2-pentafluoroéthyl)estr-4-én-3-one,
9,11α-dihydro-17β-hydroxy-6'-(1-hydroxyéthyl)-17α-(1,1,2,2,2-pentafluoroéthyl)-4'H-napht-[3',2',1':10,9,11]estr-4-én-3-one.

**26.** Préparations pharmaceutiques contenant au moins un 17α-fluoroalkylstéroïde de formule générale selon la revendication 1, ainsi qu'un véhicule pharmaceutiquement acceptable.

**27.** Préparations pharmaceutiques selon la revendication 26, contenant en outre au moins un composé à action antiestrogène.

**28.** Utilisation des 17α-fluoroalkylstéroïdes de formule générale I selon la revendication 1, pour la fabrication de médicaments.

**29.** Utilisation selon la revendication 28, en outre avec un composé à action antiestrogène